(19) Europäisches Patentamt
European Patent Office
Office européen des brevets

(11) Publication number : 0 510 952 A1

(12) # EUROPEAN PATENT APPLICATION

(21) Application number : 92303625.5

(22) Date of filing : 23.04.92

(51) Int. Cl.$^5$ : C07H 21/04, C12Q 1/68, C12Q 1/70, C12N 15/51, C12P 19/34

(30) Priority : 26.04.91 JP 191376/91

(43) Date of publication of application : 28.10.92 Bulletin 92/44

(84) Designated Contracting States : BE CH DE DK FR GB IT LI NL

(71) Applicant : IMMUNO JAPAN INC.
4-28-14-701, Ogikubo Suginami-Ku
Tokyo 167 (JP)

(72) Inventor : Okamoto, Hiroaki
3132-24, Yakushiji, Minami Kawachi Machi
Kawachi-gun, Tochigi-ken 329-04 (JP)
Inventor : Nakamura, Tetsuo
14-701, 4-chome 28, Ogikubo, Suginami-ku
Tokyo 167 (JP)

(74) Representative : Arthur, Bryan Edward
Withers & Rogers 4 Dyer's Buildings Holborn
London EC1N 2JT (GB)

(54) **Oligonucleotide primers and their application for high-fidelity detection of non-a, non-b hepatitis virus.**

(57) A non-A, non-B hepatitis virus detection system using oligonucleotide primers having nucleotide sequences corresponding to part of the viral RNA of NANB hepatitis. Oligonucleotide primers derived from viral RNA of non-A, non-B hepatitis can be used to to detect non-A, non-B hepatitis virus.

EP 0 510 952 A1

## Reference To A Related Application

The present application is a continuation-in-part of our copending U.S. Patent Application Serial No. 07/7l2,875, filed on June ll, l99l, which is incorporated by reference in its entirety.

## Introduction to the Invention

The present invention concerns high-tidelity detection of non-A, non-B hepatitis virus (hereinafter called NANB hepatitis virus) and oligonucleotide primers used in a detection system for detecting NANB hepatitis virus.

Viral hepatitis of which DNA and RNA have been elucidated include hepatitis A, hepatitis B, hepatitis D and hepatitis E. However, in spite of great efforts by scientists the world over, the causative virus of NANB hepatitis (which is mainly caused by blood bourne infection) falls in none of the above groups and has not been isolated.

In l988, Chiron Corp. reported that it had succeeded in cloning the RNA virus genome of the causative agent of NANB hepatitis (which it termed hepatitis C virus (hereinafter called HCV)) and disclosed part of the nucleotide sequence of HCV. HCV antibody detection systems based on that sequence are now being introduced for screening of blood for transfusion and for diagnosis of patients.

Detection systems for the HCV antibody have proven their partial association with NANB hepatitis; however, there remains serious problems such as insufficient detectability because only about 70% of asymptomatic carriers and chronic hepatitis patients are detected, or the detection system fails to detect the antibody in the early phase of infection, thus leaving problems yet to be solved even after development of the HCV antibody by Chiron Corp.

More than 95% of posttransfusion hepatitis cases in Japan are NANB hepatitis. There are 280,000 annual estimated cases of this disease. The course of NANB hepatitis is troublesome, with most patients becoming carriers who develop chronic hepatitis. In addition, those patients with chronic hepatitis develop liver cirrhosis and then hepatocellular carcinoma at a fairly high rate over l0 to 20 years. Therefore it is imperative to isolate the virus itself and to develop effective diagnostic reagents enabling earlier diagnosis.

As described earlier, there are significant numbers of patients with acute or chronic NANB hepatitis which ran not be diagnosed by the detection systems using Chiron's HCV antibody. NANB hepatitis virus in Japan has significantly different nucleotide sequence compared with that revealed by Chiron. For accurate diagnosis of these cases of hepatitis, detection systems having superior specificity and sensitivity is required.

## Summary of the Invention

An object of the present invention is to provide a highly sensitive detection system for NANB hepatitis virus at its gene level and oligonucleotide primers used for such system.

## Brief Description of the Drawing

Figures l-5 represent restriction maps of the nucleotide sequences of 5′ termini of NANB hepatitis virus strains HC-Jl, HC-J4, HC-J5, HC-J6 and HC-J7 respectively. Figure 6 shows the method of determination of the nucleotide sequence of 3′ termini of the strains. Solid lines show nucleotide sequences determined by clones from libraries of bacteriophage lambda gtl0, and broken lines show nucleotide sequences determined by clones obtained by PCR.

## Detailed Description of the Invention

For the purpose of elucidation of the NANB hepatitis viral gene, the inventors isolated NANB hepatitis viral RNA from human and chimpanzee carrier sera and determined the nucleotide sequence by cloning its cDNA covering the whole genome, including the noncoding region of the virus. As a result, the existence of a highly conserved region and a region with frequent displacement have been identified.

Abbreviations used in this invention are as follows: for RNA, A, G, C and U stand for adenine, guanine, cytosine and uracil respectively; for DNA, A, G and C indicate the same bases as in RNA and T stands for thymine; for polypeptides, A, R, N, D, C, E, Q, G, H, I, L, K, M, F, P, S, T, W, Y and V are respectively the amino acids of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine.

In the method described below, NANB hepatitis virus RNA under this invention was obtained and its nucleotide sequence was determined.

Five samples (HC-Jl, HC-J4, HC-J5, HC-J6, and HC-J7) were obtained from human and chimpanzee plasma. HC-Jl, HC-J5, and HC-J6 were obtained from Japanese blood donors, and HC-J7 from a Japanese hemodialysis patient with CRF (chronic renal failure), all of whom tested positive for HCV antibody. HC-J4 was obtained from the chimpanzee subjected to the challenge test but was negative for Chiron's HCV antibody previously mentioned.

RNA was isolated from each of the five plasma samples and the homology of the nucleotide sequences were compared with each other. The homology of the nucleotide sequences were compared with each other and the respective nucleotide sequences of about l900 to 2500 nucleotides of the 5′ terminus and about ll00 nucleotides of the 3′ terminus were determined (as described in the examples). The present inventors have completely studied the non-coding region, the regions coding for structural protein, and the partial regions coding for non-structural protein of all five strains.

As shown in Example l, strains HC-Jl, HC-J4, HC-J5, HC-J6 and HC-J7 had a 5′ region consisting of l885 to 255l nucleotides that contains the noncoding region consisting of at least 340 or 34l nucleotides and a region coding for the structural protein (ll49 nucleotides) followed by a region coding for the non-structural protein (see sequence lists l-5).

Concerning the 3′ terminus and 5′ terminus, as described in example 2, HC-Jl, HC-J4, HC-J5, HC-J6 and HC-J7 were found to have a region consisting of l096 nucleotides coding for the non-structural protein followed by non-coding region consisting of at least 77 nucleotides that contains the T-stretch of 3′ terminus. The 3′ terminal nucleotides are shown in sequence lists 6-l0.

When compared with the sequence of HCV disclosed in European Patent Publication No. 388,232 (by Chiron Corp.), the homology of the sequences under this invention in comparison to HCV was 95.2% for strain HC-Jl, 80.5% for strain HC-J4, 72.5% for strain HC-J6 and 7l.6% for HC-J7 in the 2477 nucleotides of the 5′ terminus including the non-coding region, and only 76.8% for HC-J7 in 1468 nucleotides. This result may suggest that strain HC-Jl may be relatively close to the strain determined by Chiron Corp. and thought to be an American type virus, but the others are different and are Japanese type viruses.

In order to detect NANB virus using polymerase chain reaction, which has high detectability both in specificity and sensitivity, methods were developed using the primers having the sequences shown in sequence lists ll-42.

The 5′ noncoding regions of viral RNA of the five strains were found to consist of 340 or 34l nucleotides, and the nucleotides are highly conserved in that region. For example, the difference in nucleotides between strains HC-Jl and HC-J4 was only eight and the difference between strains HC-Jl and HC-J7 was twenty. Strains HC-J5 and HC-J6 are thought to have the completely same sequence of 340 nucleotides.

The good conservation of nucleotide sequences in the 5′ noncoding region was surprising when there is considered that very frequent displacement was observed in the other region of viral RNA of the five strains. In addition, the upper stream of the coding region starting with ATG has comparatively small variation. Accordingly, NANB viral RNA was found to be sensitively detected independent from the variations of the strains by using oligonucleotides #23, #25, #32, #33, #36 and #48 deduced from the region as primers.

When the amino acid sequences of strains HC-Jl and HC-J4 were compared, there was higher amino acid sequence homology in the upstream region than in the downstream region, and the upstream region was hydrophilic (containing more basic amino acids like arginine). Basic amino acids tend to be found in the core protein of various viruses providing affinity with nucleic acids; therefore, this upstream region was considered to code for the core protein while the downstream region (where frequent displacement of amino acids and as many as eight glycosylation sites were found) was considered to be coding for the surface protein.

Homology of the amino acid sequences of the 5′ terminus of each strain was examined and divided into upper region and lower region. The homology of HC-Jl and HC-J4 was 96.9% and that of HC-J6 and HC-J7 was 95.3% for amino acids l-l9l throughout the region considered as core; however, homology between MC-Jl and HC-J6 and between HC-J4 and HC-J7 were only 90.6%. For amino acids 192-383 which is considered envelope protein, the homology was lower than that of the core region; the homology between HC-Jl and HC-J4 was 77.6% and between HC-J6 and HC-J7 was 7l.4%; homology between HC-Jl and HC-J6 was 60.9% and between HC-J4 and HC-J7 was 52.6%.

The homology of the 3′ terminal sequence (365 amino acids) was 87.4% between HC-Jl and HC-J4 and was 89.6% between HC-J6 and HC-J7; the homology between HC-Jl and HC-J6 was 73.7%. Homology for 39 nucleotides of the non-coding region was 64.l% for HC-Jl and HC-J4 and was 82.l% for HC-J6 and HC-J7; the homology between HC-Jl and HC-J6 was 30.8%. These results reflect the differences between the American type and Japanese type viruses and also variation among the Japanese types.

The oligonucleotide primers below (sequence lists ll to 42) are favorable to detect NANB viral genome with high sensitivity:

#23 (sequence list ll; sense; all strains).
#25 (sequence list l2; antisense; all strains).
#32 (sequence list l3; sense; all strains).
#33 (sequence list l4; sense; all strains).
#36 (sequence list l5; antisense; all strains).
#38 (sequence list l6; sense; strain HC-J5).
#41 (sequence list l7; antisense; strain HC-J5).
#48 (sequence list l8; antisense; all strains).
#50 (sequence list l9; sense; strains HC-J5, HC-J6, and HC-J7).
#5l (sequence list 20; antisense; strain HC-J5).
#54 (sequence list 2l; antisense; strains HC-J5 and HC-J7).
#55 (sequence list 22; sense; strain HC-J5).
#57 (sequence list 23; sense; strain HC-J4).
#61 (sequence list 24; sense; strain HC-J4).
#63 (sequence list 25; antisense; strain HC-J5).
#78 (sequence list 26; sense; strain HC-J4).
#80 (sequence list 27; sense; all strains).
#90 (sequence list 28; sense; strain HC-J6).
#9l (sequence list 29; sense; strain HC-Jl).
#97 (sequence list 30; sense; strain HC-J6).
#l00 (sequence list 3l; sense; strain HC-Jl).
#122 (sequence list 32; antisense; strains HC-J6 and HC-J7).
#l23 (sequence list 33; sense; strain HC-J7).
#l24 (sequence list 34; sense; strain HC-J7).
#l27 (sequence list 35; antisense; strain HC-J7).
#l28 (sequence list 36; antisense; strain HC-J6).
#l29 (sequence list 37; sense; strain HC-J7).
#l36 (sequence list 38; antisense; strain HC-J7).
#l45 (sequence list 39; sense; strain HC-J7).
#l48 (sequence list 40; sense; strain HC-Jl).
#l49 (sequence list 42; sense; strain HC-J6).
#l50 (sequence list 49; sense; strain HC-J7).

Oligonucleotides #38, #4l, #50, #5l, #54, #55, #57, #6l, #63, #78, #90, #9l, #97, #l22, #l23, #l24, #l27, #l28, #l29, #l36, #l45, #l49 and #l50 are useful for detection and identification of at least Japanese type strains.

Polynucleotides having the sequences shown in sequence lists l to l0 were found to be specific to a part of the noncoding and coding region of NANB viral RNA, as described above, and oligonucleotides were synthesized as primers based on those polynucleotides.

The present invention also applies to oligonucleotides having some displacement (e.g., resulting from mutation between strains) as far as the oligonucleotides having specific sequences to NANB hepatitis virus RNA.

In the detection of NANB hepatitis virus by PCR, more favorable effect is obtained by using primers in pairs. Examples of effective pairs are as follows: #23 and #25; #32 and #36; #33 and #48; #38 and #4l; #50 and #54; #50 and #63; #50 and #l28; #54 and #55; #l27 and #l29; #l36 and #l45.

The present invention also relates to diagnostic systems of NANB hepatitis virus using such primers.

The present invention also relates to a method of high-fidelity detection of NANB hepatitis viral RNA using an oligonucleotide primer having a nucleotide sequence specific to part of the coding region or noncoding region of NANB hepatitis virus RNA.

The present invention also relates to a method of high-fidelity detection of NANB hepatitis viral RNA using polymerase chain reaction with an oligonucleotide primer pair. Preferably, cDNA is amplified twice using two pairs of oligonucleotide primers.

Examples of application of this invention are shown below. However, this invention shall in no way be limited to those examples.

## Examples

### Example 1

5′ terminal nucleotide sequence and amino acid sequence of NANB hepatitis virus genome were determined in the following way:

(l) Isolation of RNA

RNA of the sample (HC-Jl, HC-J5, HC-J6) from plasma of a Japanese blood donor and that of the sample (HC-J7) from the plasma of the Japanese hemodialysis patient with CRF (who tested positive for HCV antibody by Ortho HCV Ab ELISA, Ortho Diagnostic System, Tokyo), and that of the sample (HC-J4) from the chimpanzee challenged with NANB hepatitis for infectivity (and negative for HCV antibody), were isolated in the following method.

l.8 ml of the plasma samples was added with l ml of Tris chloride buffer (l0 mM, pH 8.0) and centrifuged at 68 x l0$^3$ rpm for l hour. Its precipitate was suspended in Tris chloride buffer (50 mM, pH 8.0; containing 200 mM NaCl, l0 mM EDTA, 2% (w/v) sodium dodecyl sulfate (SDS), and l mg/ml proteinase K) and was incubated at 60°C for l hour. Then the nucleic acids were extracted by phenol/chloroform and precipitated by ethanol to obtain RNAs.

(2) cDNA Synthesis

After heating the RNA isolated from HC-Jl plasma at 70°C for l minute, this was used as a template; l0 units of reverse transcriptase (cDNA Synthesis System Plus, Amersham Japan) and 20 pmol of oligonucleotide primer (20 mer) were added and incubated at 42°C for l.5 hours to obtain cDNA. Primer #8 (5′- G A T G C T T G C G G A A G C A A T C A - 3′) was prepared by referring to the basic sequence shown in European Patent Application No. 883l0922.5, which is relied on and incorporated herein by reference.

(3) cDNA Was Amplified by the following Polymerase Chain Reaction (PCR)

cDNA was amplified for 35 cycles according to Saiki's method (Science (l988) 239: 487-49l) using Gene Amp DNA Amplifier Reagent (Perkin-Elmer.Cetus) on a DNA Thermal Cycler (Perkin-Elmer.Cetus).

(4) Determination of 5′ Terminal Nucleotide Sequence of HC-Jl and HC-J4 by assembling cDNA Clones

As shown in Figures l and 2, nucleotide sequences of 5′ termini of the genomes of strains HC-Jl and HC-J4 were determined by combined analysis of clones obtained from the cDNA library constructed in bacteriophage lambda gtl0 and clones obtained by amplification of HCV specific cDNA by PCR.

Fig. l shows 5′ terminus of NANB hepatitis virus genome together with cleavage site by restriction endonuclease and sequence of primers used. In Fig. l, solid lines are nucleotide sequences determined by clones from bacteriophage lambda gtl0 library while dotted lines show sequences determined by clones obtained by PCR.

A l656 nucleotide sequence of HC-Jl (spanning nt454-2109) was determined by clone ϕ4l which was obtained by inserting the cDNA synthesized with the primer #8 into lambda gtl0 phage vector (Amersham).

Primer #25 (5′- T C C C T G T T G C A T A G T T C A C G -3′) of nt824-843 was synthesized based on the ϕ41 sequence, and four clones (ϕ60, ϕ6l, ϕ66 and ϕ75) were obtained to cover the upstream sequence ntl8-843.

To determine the extreme upstream of the 5′ terminus of HC-J4, single-stranded cDNA was synthesized using antisense primer #36 (5′- A A C A C T A C T C G G C T A G C A G T -3′) of nt246-265, and then it was added with dATP tail at its 3′ terminus by terminal deoxynucleotidyl transferase, then amplified by one-sided PCR in two stages. That is, in the first stage, oligo dT primer (20-mer) and antisense primer #48 (5′- G T T G A T C C A A G A A A G G A C C C -3′) of ntl88-207 were used to amplify the dA-tailed cDNA by PCR for 35 cycles; and in the second stage, using the product of the first-stage PCR as a template, oligo dT primer (20-mer) and antisense primer #109 (2l-mer; 5′- A C C G G A T C C G C A G A C C A C T A T -3′) of ntl40-l60 were added to initiate PCR for 30 cycles. The product of PCR was subcloned to Ml3 phage vector. Six independent clones (C8962, C8968, C8970, C8974, C9034 and C9036) were obtained (each considered having complete length of 5′ terminus), and the nucleotide sequence of ntl-l7 of the respective clones was determined.

The upstream sequence of strain HC-Jl, in the region of ntl8-843, was determined by clones C2503, C2508 and C25l0 which were obtained by PCR amplification by the primers #44 (5′- G G C G A C A C T C C A C C A T G A A T -3′) and #25 (5′- T C C C T G T T G C A T A G T T C A C G -3′). Nucleotide sequence of 5′ terminus further upstream, ntl-37, was determined by l6 clones, C893l, C8932, C8935, C8937, C8942, C8944, C8949, C8950, C895l, C8954, C8955, C9023, C9026, C9030, C903l and C9032 obtained by the same method as for the HC-J4 strain.

The downstream sequence of the 5′ terminus from ntl984-2560 of HC-Jl was determined by 3 clones of Cll444, Cll450 and Cll45l obtained by PCR amplification for 30 cycles by primers #l48 (5′-T G C A C C T G G A T G A A C T C A A C-3′) and #l46 (5′-A G T A G C A T C A T C C A C A A G C A-3′).

The downstream sequence from nt738 to l900 of strain HC-J4 (having ll63 nucleotides) was determined by three clones of C282l, C3l73 and C3l92 which were obtained by PCR amplification by primers #30 (5′- C T C A T G G G G T A C A T T C C G C T -3′) and #42 (5′- T C G G T C G T C C C C A C C A C A A C -3′).

Further downstream sequence from ntl860-2560 of strain HC-J4 was determined by 3 clones of Cll462, Cll463 and Cll464 obtained by PCR amplification by primers #57 (5′-T A T T G C T T C A C C C C A A G C C C-3′) and #l46 above.

From the analysis described above, full nucleotide sequences of 5′ termini of the genomes of HC-Jl, HC-J4 and HC-J5 were determined as shown below.

From the analysis described above, full nucleotide sequences of 5′ termini of genomes of HC-Jl and HC-J4 were determined as shown in figure l and figure 2 (see sequence lists l and 2).

5) Determination of 5′ Terminal Nucleotide Sequence Of HC-J5 by Assembling cDNA Clones

The nucleotide sequence of the 5′ terminus of strain HC-J5 was determined by analysis of clones obtained by PCR amplification as shown in figure 3.

Isolation of RNA from HC-J5, and determination of its sequence, was conducted in the same manner as described above.

Sequences in the range of nt2l-l886 of the RNA were determined by respective clones obtained by amplification by PCR utilizing each pair of primers shown below:

nt24-265

#32 (5′- A C T C C A C C A T A G A T C A C T C C -3′)
#36 (5′- A A C A C T A C T C G G C T A G C A G T -3′)
Clones: C3534, C3536, C3537.

nt63-508

#33 (5′- T T C A C G C A G A A A G C G T C T A G -3′)
#5l (5′- G A C C G C T C C G A A G T C T T C C T -3′)
Clones: C4658, C4659, C4660.

nt467-843

#23 (5′- T A G A T T G G G T G T G C G C G C G A -3′)
#25 (5′- T C C C T G T T G C A T A G T T C A C G -3′)
Clones: C245l, C2453, C2454.

nt732-934

#50 (5′- G C C G A C C T C A T G G G G T A C A T -3′)
#63 (5′- C T G G T G T T C T T A A C C T G G A C -3′)
Clones: C5592, C5593, C5594.

nt867-l354

#55 (5′- A T T T T C T T G C T G G C C C T G C T -3′)
#54 (5′- A T C G C G T A C G C C A G G A T C A T -3′)
Clones: C5l64, C5303, C533l.

ntl240-l880

#38 (5′- T G C A A T T G T T C T A T C T A C C C -3′)
#4l (5′- C A G G G C T T G G G G T G A A G C A A -3′)
Clones: C3722, C3748, C3753.

ntl842-l976

#4 (5′- A G T G T G T G T G G T C C G G T A T A -3′)
#5 (5′- C G G T G G C C T G G T A T T G T T A A -3′)
Clones: C3952, C3969, C3990.

Three cDNA clones (C8995, C8997, C8998) were obtained in the same way as in example l(4) to determine the nucleotide sequence from ntl-l60 of further upstream of 5′ terminus.

From the analysis described above, the full nucleotide sequence of 5′ terminus of the genome of strain HC-J5 was determined, as shown in sequence list 3.

The non-coding region of the 5′ terminus of HC-J5 was found to have 340 nucleotides, lacking a cytosine out of 5 cytosines from nt6-l0 of HC-Jl.

6) Determination of 5′ Terminal Nucleotide Sequence Of HC-J6 by assembling cDNA Clones

The nucleotide sequence of the 5′ terminus of strain HC-J6 was determined by analysis of clones obtained by PCR amplification as shown in figure 4.

Isolation of RNA from HC-J6 and determination of its sequence was conducted in a manner as described above.

Sequences in the range of nt24-255l of the RNA were determined by respective clones obtained by amplification by PCR utilizing each pair of primers shown below.

nt24-826

#32 (5′- A C T C C A C C A T A G A T C A C T C C -3′)
#l22 (5′-A G G T T C C C T G T T G C A T A A T T -3′)
Clones: C9397, C9388, C9764

nt732-l907

#50 (5′- G C C G A C C T C A T G G G G T A C A T -3′)
#l22 (5′-T C G G T C G T G C C C A C T A C C A C -3′)
Clones: C93l6, C9752, C9l53

ntl847-257l

#l49 (5′-T C T G T G T G T G G C C C A G T G T A-3′)
#l46 (5′-A G T A G C A T C A T C C A C A A G C A-3′)
Clones: Cll62l, Cll624, Cll655

Further upstream of the 5′ terminus, from nt l-l60, was determined by the following l3 clones in the same way as example l(4): C9577, C9579, C958l, C9587, C9590, C959l, C9595, C9606, C9609, C96l5, C96l6, C96l9.

From the analysis described above, the full nucleotide sequence of the 5′ terminus of the genome of strain HC-J6 was determined as shown in sequence list 4.

The noncoding region of the 5′ termimus of HC-J6 was found to have 340 nucleotides lacking a cytosine out of 5 cytosines from nt6-10 of HC-Jl.

7) Determination of 5′ Terminal Nucleotide Sequence Of HC-J7 by assembling cDNA Clones

The nucleotide sequence of the 5′ terminus of strain HC-J7 was determined by analysis of clones obtained by PCR amplification as shown in figure 5.

Isolation of RNA from strain HC-J7 and determination of its sequence was made in a manner as described above. Sequences in the range of nt24-2498 of the RNA were determined by respective clones obtained by amplification by PCR utilizing each pair of primers shown below:

nt24-826

#32 (5′- A C T C C A C C A T A G A T C A C T C C -3′)
#l22 (5′-A G G T T C C C T G T T G C A T A A T T -3′)
Clones: Cl062l, Cl0622, Cl0623.

nt732-l354

#50 (5′- G C C G A T C T C A T G G G G T A C A T -3′)
#54 (5′- A T C G C G T A C G C C A G G A T C A T -3′)
Clones: Cl046l, Cl0463, Cl06l5.

ntl309-l625

#l29 (5′-C G C A T G G C A T G G G A T A T G A T-5′)
#l27 (5′-A T G T G C C A A C T G C C G T T G G T-3′)
Clones: Clll83, Clll84, Clll85

ntl566-l888

#l45 (5′-C A G G A T A T C A G T C T A A T C A A-3′)
#l36 (5′-A C T G G G C T G G G A G T G A A A C A-3′)
Clones: Clll36l, Clll364, Cll374

ntl833-25l8

#l50 (5′-A T C G T C T C G G C T A A G A C G G T-3′)
#l46 (5′-A G T A G C A T C A T C C A C A A G C A-3′)
Clones: Cll535, Cll540, Cll566

Further upstream of the 5′ terminus, from nt l-l60, was determined by the following 8 clones in the same way as example l(4): Cl05l3, Cl05l5, Cl052l, Cl0554, Cl0558, Cl0568, Cll23l, Cll232.

From the analysis described above, the full nucleotide sequence of the 5′ terminus of the genome of strain HC-J7 was determined as shown in sequence list 5.

The 5′ terminal sequences of HC-Jl, HC-J4, HC-J5, HC-J6, and HC-J7 disclosed in the examples were different from the HCV sequence in European Patent Application Publication No. 388,232.

Example 2.

3′ terminal nucleotide sequence and amino acid sequence of NANB hepatitis virus genome were determined in the following way:

(l) Determination of 3′ Terminal Nucleotide Sequence By assembling cDNA Clones

As shown in figure 6, nucleotide sequences of 3′ termini of genomes of strains HC-Jl, HC-J4, HC-J5, HC-J6, and HC-J7 were determined by analysis of clones obtained by amplification of HCV specific cDNA by PCR.

The 938 nucleotide sequence of ntl-938 of strains HC-Jl, HC-J4, HC-J5, HC-J6, and HC-J7 were determined by analysis of amplification products obtained by PCR using primer #80 (5′-G A C A C C C G C T G T T T T G A C T C-3′) and #60 (5′-G T T C T T A C T G C C C A G T T G A A-3′).

Obtained clones are shown below:
HC-Jl: C739l, C7343, C7377;
HC-J4: C5584, C5585, C5586;
HC-J5: C72l2, C7257, C726l;
HC-J6: C9760, C9234, C976l;
HC-J7: C9928, Clll25, Clll4l.

Nucleotide sequences of 3′ termini downstream from nt939 were determined in the manner shown below:

RNA was extracted from each sample in the manner described in example l, followed by addition of poly(A) to 3′ terminus of RNA using poly(A)-polymerase. cDNA was synthesized using oligo$(dT)_{20}$ as a primer, and used as a template in PCR.

lst stage PCR was performed using sense primer specific to each strain and oligo$(dT)_{20}$ as antisense primer. Then 2nd stage PCR was performed using cDNA obtained by lst stage PCR using sense primer specific to each strain but downstream of that used in lst stage PCR and oligo$(dT)_{20}$ as antisense primer. After smoothening both ends of PCR product obtained by two stage PCR utilizing $T_4$DNA polymerase and phosphorylating the 5′ terminus utilizing $T_4$ polynucleotide kinase, the nucleotide sequence was determined by subcloning to Hinc II site of Ml3mpl9 phage vector.

Each pair of primer utilized in PCR and obtained clones are shown below:

HC-Jl

#l00 (5′-A A G G C T G C C A T A T G T G G C A A-3′)
#9l (5′-G C C A T A T G T G G C A A G T A C C T-3′)
Clones: C9707, C97l4, C97l9, C9724, C9726, C9730, C9737, C9738, C974l, C9742, C9746, C9925, C9936, C9943, C9945, C9949.

HC-J4

#6l (5′-T T G C G A G T C T G G A G A C A T C G-3′)
#78 (5′-T G T C C G C G C T A A G C T A C T G T-3′)
Clones: C876l, 8764, 8776, C8784, C8796, C8800, C8803, C88ll, C88l2, C88l9, C8825, C8849, C885l.

HC-J5

#97 (5′A G T C A G G G C G T C C C T C A T C T′3′)
#90 (5′-G C C G T T T G C G G C C G A T A T C T-3′)
Clones: Cl0820, Cl0827, l0829, l0843, l0848, l0849, l0864, l0865, l0872.

HC-J6

#97 (5′-A G T C A G G G C G T C C C T C A T C T-3′)
#90 (5′-G C C G T T T G C G G C C G A T A T C T-3′)
Clones: Cl0820, Cl0827, Cl0829, Cl0843, Cl0844, Cl0848, Cl0849, Cl0864, Cl0865, Cl0872.

HC-J7

#l23 (5′C T T A G A G C G T G G A A G A G T C G-3′)
#l24 (5′-G C C A T C T G T G G C C G T T A C C T-3′)
Clones: Cl080l, Cl0804, Cl0807, Cl0809, Cl08ll, Cl08l2, Cl08l4, Cl08l5, Cl08l8

From the analysis described above, the full nucleotide sequences of 3′ terminal sequences of strains HC-Jl, HC-J4, HC-J5, HC-J6 and HC-J7 are shown in sequence sequence list 6-10.

The 3′ terminal sequences of strains HC-Jl, HC-J4, HC-J5, HC-J6 and HC-J7 disclosed in the examples, to a certain extent, overlapped HCV sequence of European Patent Application Publication No. 388,232 in nt 7938-8866 (929 nucleotides) but were different from it.

Example 3 - Synthesis or primers and the establishment of the detection system based on the 5′ noncoding region and the core protein coding region.

(l) Synthesis of oligonucleotide primers.

Oligonucleotide primers (20 mer) were synthesized based on the 5′ noncoding region sequences and the core protein coding region of strains HC-Jl and HC-J4 determined in Example l(4). Oligonucleotide primer of HCV was also synthesized according to the nucleotide sequence disclosed in the European Patent Application No. 883l0922.5 previously described. The model 3808 DNA Synthesizer (Applied Biosystems Japan) was used for such synthesis.

The number of primers synthesized were 20 (#3, 4, 5, 6, 9, l0, ll, l2, l6, l7, 2l, 22, 23, 25, 32, 33, 34, 35, 36 and 48), and the position from the 5′ terminus and nucleotide sequence for each of them is shown in Table l.

(2) Isolation of NANB hepatitis viral RNA from a sample.

l ml of a plasma sample was centrifuged on a model TL-l00 (Beckman) ultracentrifuge at 9 x $l0^4$ rpm for l5 minutes and the precipitate thus obtained was suspended in buffer (containing 200 mM NaCl, l0 mM EDTA, 2% (w/v) sodium doderyl sulfate (SDS) and proteinase K (l mg/ml)) for incubation at 60°C for l hour.

Nucleic acids were extracted twice by using the same volume of phenol/chloroform and precipitated in ethanol at -20°C for over 3 hours. The precipitate was suspended in 70% ethanol for centrifugation and the precipitate was dissolved in 5 µl of distilled water after lyophilization.

(3) cDNA synthesis.

RNA extracted from a plasma sample in (2) above was denatured by heating at 70°C for l minute and cooled on ice before synthesis of cDNA. cDNA was synthesized by reverse transcription. 100 pmol each of antisense primers #5, 6, ll, l2, l6, l7, 25, 35, 36 and 48 were added with 4 kinds of deoxyribonucleoside 5′-triphosphates (Takara, Japan), l0 units of RNase Inhibitor (Takara, Japan) and l0 units of Reverse transcriptase AMV (Boehringer Mannheim, Germany), and incubated at 42°C for 90 minutes in a buffer (containing l0 µl of Tris chloride (50 mM, pH 8.4), 8 mM $MgCl_2$, 30 mM KCl and l mM dithiothreitol) to synthesize cDNA. cDNA thus obtained was purified by phenol/chloroform extraction.

(4) Amplification by PCR.

PCR was carried out using DNA Thermal Cycler (Perkin-Etmer Cetus) and DNA Amplification Reagent Kit (Perkin-Elmer Cetus) by the well-known method of Saiki et al. (l988). The reaction cycle of denaturalization (one minute at 94°C), annealing of primers (l.5 minutes at 55°C), and amplification of primers (3 minutes at 72°C) was repeated 35 times.

The PCR product was electrophoresed in a mixed agarose gel of l-l.5% Nusieve and l-l.5% Seakem (FMC), and, after staining with ethidium bromide, its bands were confirmed by ultraviolet radiation.

(5) Amplification by second-stage PCR.

The product obtained by the first pair of primers (#32 and #36, for example) by PCR can be subjected to second-stage PCR if necessary. As primers for such PCR, a pair of primers of nucleotides for regions within those of the first pair of primers (#33 and #48, for example) were chosen and PCR reaction cycle was repeated 30 times for 5 µl of the product obtained in the first-stage PCR. Reaction conditions for each cycle was denaturalization (l minute at 94°C), annealing (l.5 minutes at 55°C), and amplification (2 minutes at 72°C). The product obtained in the second-stage PCR was electrophoresed and analyzed in the method described in (4) above.

Example 4 - Selection of pairs of primers effective for detection of NANB hepatitis virus by PCR.

Results of PCR tests with two pairs of primers for 10 samples determined positive for HCV antibody (plasma samples nos. l, 3, 5, 7 and 9 from Japanese blood donors and serum samples nos. 2, 4, 6, 8 and l0 from NANB hepatitis patients) are shown in Table 2.

PCR amplification was tried for l0 target nucleotide sequence regions; 2 regions each from NS5, NS3, and E-NSl and its upstream (NS=nonstructural region, E-NSl-=nucleotide region bridging the envelope region and nonstructural region number l) referred to in Chiron's European Application), 2 regions each from the core region and 5′ noncoding region identified under this invention.

As a result, when two pairs of primers from the 5′ noncoding region (#32/#36 and #33/#48) and one pair of primers from the core region (#23/#25) were used, expected sizes of NANB cDNA bands (242 bp, l45 bp and 377 bp) for respective regions were detected.

In the other 7 regions, however, only 2 to 9 out of l0 samples could successfully be amplified, although the presence of RNA itself was confirmed in each sample. It was therefore concluded that pairs of primers #32/#36 and #33/#48 from the 5′ noncoding region, and #23/#25 from the core region, were widely effective for detection of HCV RNA.

In some cases, single-stage PCR is sufficient for detection of NANB hepatitis viral RNA. However, to enhance sensitivity, two-stage PCR is recommended.

For example, samples which did not show the expected band of 242 bp when their cDNA was synthesized using #36 primer and amplified by PCR with primers #36 and #32 (first-stage PCR) were then subjected to the second-stage PCR using primers #33 and #48 and the first-stage PCR product as a template (second-stage PCR).

Example 5

32 samples from chronic NANB hepatitis patients, 10 samples from chronic hepatitis B patients, and 12 samples from blood donors with normal ALT levels were tested for NANB hepatitis virus RNA by PCR. Results are shown in Table 3.

Preliminary test of 32 samples from NANB hepatitis patients showed 20 samples positive and I2 samples negative for anti-HCV. All I0 samples from hepatitis B patients and I2 samples from blood donors with normal ALT levels were negative for anti-HCV.

For the 20 samples out of the 32 total samples from NANB hepatitis patients which tested positive for anti-HCV, RNA was detected in 15 samples by the first-stage PCR and the remaining 5 by the second-stage PCR. Thus I00% of the samples (which tested positive for anti-HCV) tested positive by PCR.

Out of the I2 samples from NANB hepatitis patients which tested negative for anti-HCV, 7 samples by the first-stage PCR and 4 samples by the second-stage PCR (or 92%) turned out to be positive for NANB hepatitis viral RNA. All I0 hepatitis B cases and all I2 blood donor cases with normal ALT levels subjected to the test (so far as the second-stage PCR) were negative for the viral RNA. From these data, NANB hepatitis RNA detection system using oligonucleotide as primers has proven its excellent performance, and its two-stage PCR system in particular has proven its superb performance both in sensitivity (more than 50% higher than anti-HCV and detecting as much as 96.9% of NANB hepatitis viral RNA) and specificity.

Example 6 Sensitivity of the detection system for NANB hepatitis virus by cDNA/two-stage PCR.

Sensitivity of NANB hepatitis virus detection system by cDNA/two-stage PCR under this invention is described below. Results are shown in Table 4.

I0-fold serially diluted samples of plasma (having known infectious unit of $I0^7$ CID/ml) were prepared and tested 3 times. In the first-stage PCR, the expected band of 242 bp was confirmed for I00 CID/ml in two tests and as low as I0 CID/ml in the remaining one test.

In the second-stage PCR, the expected band of I45 bp was confirmed for I0 CID/ml in two tests and as low as I CID/ml in one test. No band was detected for concentrations less than I CID/ml or for negative samples.

Average titer of NANB hepatitis patients is estimated to be $I0^2$-$I0^4$ and the described detection system is considered to give clinically significant sensitivity for diagnosis of NANB Hepatitis patients.

The present invention thus provides a highly sensitive and specific detection system for NANB hepatitis virus. Accordingly, this invention will become instrumental in accurate diagnosis of hepatitis patients and screening of donor blood for prevention of posttransfusion hepatitis.

The present invention further concerns a method of detecting non-A, non-B hepatitis virus comprising:

(I) synthesizing cDNA from viral RNA;

(2) amplifying said cDNA by PCR in a first stage to produce a product;

(3) amplifying the product by PCR in a second stage. The amplifying is carried out by using at least one oligonucleotide primer. The primer in step (I) may be a pair of primers and the primer in step (2) may be a pair of primers from a region within the pair of primers in step (I).

The present invention also concerns diagnostic test kits for detecting NANB in biological samples, including for example blood and serum samples. The test kit includes (I) at least one primer derived from the nucleotide sequence disclosed above, (2) dATP, dTTP, dGTP, and dCTP; and (3) heat stable DNA polymerase. Kits suitable for diagnosis of NANB and containing the appropriate reagents are constructed by packaging the appropriate materials, including the primer in suitable containers, along with the remaining reagents and materials required, as well as a suitable set of instructions for conducting the test.

Further variations and modifications of the invention will become apparent to those skilled in the art from the foregoing and are intended to be encompassed by the claims appended hereto.

Japanese Patent Application No. 191376/91, filed on April 26, 1992, is relied on and incorporated by reference.

Table 1

| Primers | Nucleotide Position | Nucleotide Sequences |
| --- | --- | --- |
| #3 | 126−145 | AAACCTTGCGGTATTGTGCC |
| #4 | 153−172 | AGTGTGTGTGGTCCGGTATA |
| #5 | 268−287 | CGGTGGCCTGGTATTGTTAA |
| #6 | 303−322 | GAGTTCATCCAGGTACAACC |
| #9 | 6427−6446 | AGATGGCTTTGTACGACGTG |
| #10 | 6490−6509 | TCCAATACTCACCAGGACAG |
| #11 | 6761−6780 | CACAGCTAGTTGTCAGTACG |
| #12 | 6786−6805 | TTGATGTAGCAAGTGAGGGT |
| #16 | 4029−4048 | CTGGTGACAGCAGCTGTAAA |
| #17 | 4061−4080 | TGAAGAGGAGGGTTTGGCTA |
| #21 | 3669−3688 | TATTGCCTGTCAACAGCCTG |
| #22 | 3759−3778 | CGAGAGTTCGATGAGATGGA |
| #23 | 450−469 | TAGATTGGGTGTGCGCGCGA |
| #25 | 807−826 | TCCCTGTTGCATAGTTCACG |
| #32 | 7−26 | ACTCCACCATAGATCACTCC |
| #33 | 46−65 | TTCACGCAGAAAGCGTCTAG |
| #34 | 475−494 | AACACTTCCGAGCGGTCGCA |
| #35 | 568−587 | TTGCCATAGAGGGGCCAAGG |
| #36 | 229−248 | AACACTACTCGGCTAGCAGT |
| #48 | 171−190 | CTTGATCCAAGAAAGGACCC |

Table 2: Detection of NANB hepatitis viral RNA in HCV antibody positive samples by PCR using various sets of primers.

| Primers / Sample No. | 5' noncoding Region | | presumable core gene | | E-NS1 | | NS3 | | NS5 | |
|---|---|---|---|---|---|---|---|---|---|---|
| | #32 / #36 (242bp) | #33 / #48 (145bp) | #23 / #25 (377bp) | #34 / #35 (113bp) | #3 / #6 (197bp) | #4 / #5 (135bp) | #21 / #17 (412bp) | #22 / #16 (290bp) | #9 / #12 (379bp) | #10 / #11 (291bp) |
| 1 | + | + | + | + | + | + | + | + | + | + |
| 2 | + | + | + | + | − | + | + | + | + | − |
| 3 | + | + | + | + | − | + | + | + | + | − |
| 4 | + | + | + | + | − | + | − | + | + | − |
| 5 | + | + | + | + | − | − | − | + | + | − |
| 6 | + | + | + | + | − | + | + | + | + | − |
| 7 | + | + | + | + | − | − | − | + | + | − |
| 8 | + | + | + | + | − | − | − | + | + | − |
| 9 | + | + | + | − | − | + | − | − | − | − |
| 10 | + | + | + | − | − | + | − | − | − | − |

Samples Nos. 1, 3, 5, 7 and 9 are taken from blood donors, and samples Nos. 2, 4, 6, 8 and 10 are taken from chronic NANB hepatitis patients.

Table 3: NANB hepatitis viral RNA detection by cDNA/two-stage PCR. One pair each of primers #32 and 36, and #33 and #48 was used for the first-stage and second-stage PCR respectively.

| Source of Samples | Total number of Samples | Number of samples positive for anti-HCV by ORTHO's EIA | Number of samples positive for NANB hepatitis viral RNA | | |
|---|---|---|---|---|---|
| | | | 1st-stage PCR | 2nd-stage PCR | Total (%) |
| Chronic NANB hepatitis | 32 | 20 (62.5%) | 22 | 9 | 31 (96.9%) |
| Chronic hepatitis B | 10 | 0 ( 0 ) | 0 | 0 | 0 ( 0 ) |
| Blood donors with normal ALT level | 12 | 0 ( 0 ) | 0 | 0 | 0 ( 0 ) |

Table 4: Detection of NANB hepatitis viral RNA by two-stage PCR in samples with known infectivity titers. One pair each of primers #32 and #36, and #33 and #48 were used for the first-stage and second-stage respectively.

| Test | PCR | *Serial Dilution (CID/ml) | | | | | | | Control (Negative) |
|---|---|---|---|---|---|---|---|---|---|
| | | $\times 10^2$ ($10^5$) | $\times 10^3$ ($10^4$) | $\times 10^4$ ($10^3$) | $\times 10^5$ ($10^2$) | $\times 10^6$ (10) | $\times 10^7$ 1 | $\times 10^8$ 0.1 | |
| First Test | 1st-stage | +++ | +++ | ++ | + | - | - | - | - |
| | 2nd stage | NT | NT | NT | + | + | - | - | - |
| Second Test | 1st-stage | +++ | +++ | ++ | + | +/- | - | - | - |
| | 2nd-stage | NT | NT | NT | + | + | + | - | - |
| Third Test | 1st-stage | +++ | +++ | ++ | + | - | - | - | - |
| | 2nd-stage | NT | NT | NT | + | + | - | - | - |

*Same plasma negative for HCV antibody and HCV RNA was used as diluent and Control. CID means Chimpanzee Infectious Dose.

Sequence list No.1

Length: 2540

Type: nucleic acid

Strandedness: single

Topology: linear

Molecular Type: cDNA to genomic RNA

```
GCCAGCCCCC TGATGGGGGC GACACTCCAC CATGAATCAC TCCCCTGTGA GGAACTACTG   60
TCTTCACGCA GAAAGCGTCT AGCCATGGCG TTAGTATGAG TGTCGTGCAG CCTCCAGGAC  120
CCCCCCTCCC GGGAGAGCCA TAGTGGTCTG CGGAACCGGT GAGTACACCG GAATTGCCAG  180
GACGACCGGG TCCTTTCTTG GATAAACCCG CTCAATGCCT GGAGATTTGG GCGCGCCCCC  240
GCAAGACTGC TAGCCGAGTA GTGTTGGGTC GCGAAAGGCC TTGTGGTACT GCCTGATAGG  300
GTGCTTGCGA GTGCCCCGGG AGGTCTCGTA GACCGTGCAC CATGAGCACG ATTCCCAAAC  360
CTCAAAGAAA AACCAAACGT AACACCAACC GTCGCCCACA GGACGTCAAG TTCCCGGGTG  420
GCGGTCAGAT CGTTGGTGGA GTTTACTTGT TGCCGCGCAG GGGCCCTAGA TTGGGTGTGC  480
GCGCGACGAG GAAGACTTCC GAGCGGTCGC AACCTCGAGG TAGACGTCAG CCTATCCCCA  540
AGGTGCGTCG GCCCGAGGGC AGGACCTGGG CTCAGCCCGG GTACCCTTGG CCCCTCTATG  600
GCAATGAGGG CTGCGGGTGG GCGGGATGGC TCCTGTCTCC CCGTGGCTCT CGGCCTAGTT  660
GGGGCCCCAC GGACCCCCGG CGTAGGTCGC GCAATTTGGG TAAGGTCATC GATACCCTCA  720
CGTGCGGCTT CGCCGACCTC ATGGGGTACA TACCGCTCGT CGGCGCCCCT CTTGGAGGCG  780
CTGCCAGGGC CCTGGCGCAT GGCGTCCGGG TTCTGGAAGA CGGCGTGAAC TATGCAACAG  840
GGAACCTTCC TGGTTGCTCT TTCTCTATCT TCCTTCTGGC CCTGCTCTCT TGCCTGACTG  900
TGCCCGCTTC AGCCTACCAA GTGCGCAACT CCACAGGGCT TTATCATGTC ACCAATGATT  960
GCCCTAACTC GAGTATTGTG TACGAGGCGC ACGATGCCAT CCTGCATACT CCGGGGTGTG 1020
TCCCTTGCGT TCGCGAGGGC AACGTCTCGA GGTGTTGGGT GGCGATGACC CCCACGGTAG 1080
CCACCAGGGA CGGCAAACTC CCCGCGACGC AGCTTCGACG TCACATCGAT CTGCTTGTCG 1140
```

GGAGCGCCAC CCTCTGTTCG GCCCTCTACG TGGGGGATCT GTGCGGGTCC GTCTTCCTTA 1200
TTGGTCAACT GTTTACCTTC TCTCCCAGGC GCCACTGGAC AACGCAAGGC TGCAATTGTT 1260
CTATCTACCC CGGCCATATA ACGGGTCATC GCATGGCATG GGATATGATG ATGAACTGGT 1320
CCCCTACGGC GGCGTTGGTA ATGGCTCAGC TGCTCCGGAT CCCACAAGCC ATCTTGGATA 1380
TGATCGCTGG TGCTCACTGG GGAGTCCTGG CGGGCATAGC GTATTTCTCC ATGGTGGGGA 1440
ACTGGGCGAA GGTCCTGGTA GTGCTGTTGC TGTTTGCCGG CGTCGACGCG GAAACCATCG 1500
TCTCCGGGGG ACAAGCCGCC CGCGCCATGT CTGGACTTGT TAGTCTCTTC ACACCAGGCG 1560
CTAAGCAGAA CATCCAGCTG ATCAACACCA ACGGCAGTTG GCACATCAAT AGCACGGCCT 1620
TGAACTGCAA TGAAAGCCTT AACACCGGCT GGTTAGCAGG GCTTATCTAT CAACACAAAT 1680
TCAACTCTTC GGGCTGTCCC GAGAGGTTGG CCAGCTGCCG ACGCCTTACC GATTTTGACC 1740
AGGGCTGGGG CCCTATCAGT CATGCCAACG GAAGCGGCCC CGACCAACGC CCCTATTGTT 1800
GGCACTACCC CCCAAAACCT TGCGGTATCG TGCCCGCAAA GAGCGTATGT GGCCCGGTAT 1860
ATTGCTTCAC TCCCAGCCCC GTGGTGGTGG GAACGACCGA CAGGTCGGGC GCGCCTACCT 1920
ACAACTGGGG TGCAAATGAC ACGGACGTCT TCGTCCTCAA CAACACCAGG CCACCGCTGG 1980
GCAATTGGTT CGGTTGCACC TGGATGAACT CAACTGGATT CACCAAGGTA TGCGGAGCGC 2040
CTCCTTGTGT GATTGGAGGG GGGGGCAACA ACACCCTGCA CTGCCCCACT GATTGTTTCC 2100
GCAAGCATCC GGAAGCCACA TACTCTCGGT GCGGCTCTGG TCCCTGGATC ACACCCAGAT 2160
GCCTGGTCGA CTATCCATAT AGGCTTTGGC ATTACCCTTG TACCATCAAC TATACCATTT 2220
TTAAAGTTAG GATGTACGTG GGAGGGGTCG AGCACAGGCT GGATGCTGCC TGCAACTGGA 2280
CGCGGGGCGA ACGTTGCGAT CTGGAAGATA GGGACAGGTC CGAGCTCAGC CCGTTGCTGC 2340
TGTCCACCAC GCAGTGGCAG GTCCTTCCGT GTTCATTCAC GACCCTGCCA GCCTTGTCCA 2400
CCGGCCTCAT CCACCTCCAC CAGAACATTG TGGACGTGCA GTACCTGTAC GGGGTGGGGT 2460
CAAGCATCGC GTCCTGGGCC ATCAAGTGGG AGTACGTCGT TCTCCTGTTC CTTCTGCTTG 2520
CAGACGCGCG CGTCTGCTCC 2540

Sequence list No.2

Length: 2540

Type: nucleic acid

Strandedness: single

Topology: linear

Molecular Type: cDNA to genomic RNA

```
GCCAGCCCCC GATTGGGGGC GACACTCCAC CATAGATCAC TCCCCTGTGA GGAACTACTG   60
TCTTCACGCA GAAAGCGTCT AGCCATGGCG TTAGTATGAG TGTCGTGCAG CCTCCAGGAC  120
CCCCCCTCCC GGGAGAGCCA TAGTGGTCTG CGGAACCGGT GAGTACACCG GAATTGCCAG  180
GACGACCGGG TCCTTTCTTG GATCAACCCG CTCAATGCCT GGAGATTTGG GCGTGCCCCC  240
GCGAGACTGC TAGCCGAGTA GTGTTGGGTC GCGAAAGGCC TTGTGGTACT GCCTGATAGG  300
GTGCTTGCGA GTGCCCCGGG AGGTCTCGTA GACCGTGCAC CATGAGCACG AATCCTAAAC  360
CTCAAAGAAA AACCAAACGT AACACCAACC GCCGCCCACA GGACGTCAAG TTCCCGGGCG  420
GTGGTCAGAT CGTTGGTGGA GTTTACCTGT TGCCGCGCAG GGGCCCCAGG TTGGGTGTGC  480
GCGCGACTAG GAAGACTTCC GAGCGGTCGC AACCTCGTGG ATGGCGACAA CCTATCCCCA  540
AGGCTCGCCG ACCCGAGGGC AGGGCCTGGG CTCAGCCCGG GTACCCTTGG CCCCTCTATG  600
GCAATGAGGG CTTGGGGTGG GCAGGATGGC TCCTGTCACC CCGCGGCTCC CGGCCTAGTT  660
GGGGCCCCAC GGACCCCCGG CGTAGGTCGC GTAACTTGGG TAAGGTCATC GATACCCTTA  720
CATGCGGCTT CGCCGATCTC ATGGGGTATA TTCCGCTCGT CGGCGCCCCC CTAGGGGGCG  780
CTGCCAGGGC CTTGGCACAC GGTGTCCGGG TTCTGGAGGA CGGCGTGAAC TATGCAACAG  840
GGAACTTGCC CGGTTGCTCT TTCTCTATCT TCCTCTTGGC TTTGCTGTCC TGTTTGACCA  900
TCCCAGCTTC CGCTTATGAA GTGCGCAACG TGTCCGGGAT ATACCATGTC ACGAACGACT  960
GCTCCAACTC AAGCATTGTG TATGAGGCAG CGGACATGAT CATGCATACT CCCGGGTGCG 1020
TGCCCTGCGT TCGGGAGGAC AACAGCTCCC GTTGCTGGGT AGCGCTCACT CCCACGCTCG 1080
CGGCCAGGAA TGCCAGCGTC CCCACTACGA CAATACGACG CCACGTCGAC TTGCTCGTTG 1140
GGGCGGCTGC TTTCTGCTCC GCTATGTACG TGGGGGATCT CTGCGGATCT GTTTTCCTCG 1200
```

TCTCCCAGCT GTTCACCTTC TCGCCTCGCC GGCATGAGAC AGTGCAGGAC TGCAACTGCT 1260
CAATCTATCC CGGCCATTTA TCAGGTCACC GCATGGCTTG GGATATGATG ATGAACTGGT 1320
CACCTACAAC AGCCCTAGTG GTGTCGCAGT TGCTCCGGAT CCCACAAGCT GTCGTGGACA 1380
TGGTGGCGGG GGCCCACTGG GGAGTCCTGG CGGGCCTTGC CTACTATTCC ATGGTAGGGA 1440
ACTGGGCTAA GGTCCTGATT GTGGCGCTAC TCTTCGCCGG CGTTGACGGG GAGACCTACA 1500
CGTCGGGGGG GGCGGCCAGC CACACCACCT CCACGCTCGC GTCCCTCTTC TCACCTGGGG 1560
CGTCTCAGAG AATCCAGCTT GTGAATACCA ACGGCAGCTG GCACATCAAC AGGACTGCCC 1620
TAAACTGCAA TGACTCCCTC CACACTGGGT TCCTTGCCGC GCTGTTCTAC ACACACAGGT 1680
TCAACTCGTC CGGGTGCCCG GAGCGCATGG CCAGCTGCCG CCCCATTGAC TGGTTCGCCC 1740
AGGGATGGGG CCCCATCACC TATACTGAGC CTGACAGCCC GGATCAGAGG CCTTATTGCT 1800
GGCATTACGC GCCTCGACCG TGTGGTATCG TACCCGCGTC GCAGGTGTGT GGTCCAGTGT 1860
ATTGCTTCAC CCCAAGCCCT GTTGTGGTGG GGACCACCGA TCGTTCCGGT GTCCCTACGT 1920
ATAGCTGGGG GGAGAATGAG ACAGACGTGA TGCTTCTCAA CAACACGCGT CCGCCACAAG 1980
GCAACTGGTT CGGCTGTACA TGGATGAATA GTACTGGGTT CACTAAGACG TGCGGAGGCC 2040
CCCCGTGTAA CATCGGGGGG GTCGGTAACC ACACCTTGAC CTGCCCCACG GACTGCTTCC 2100
GGAAGCACCC CGAGGCTACT TACACAAAAT GTGGCTCGGG GCCCTGGTTG ACACCTAGGT 2160
GCCTAGTAGA CTACCCATAC AGGCTCTGGC ACTACCCCTG CACTTTCAAT TTTTCCATCT 2220
TTAAGGTTAG GATGTATGTG GGGGGCGTGG AGCACAGGCT CAATGCCGCA TGCAATTGGA 2280
CTCGAGGAGA GCGCTGTAAC TTGGAGGACA GGGACAGGTC AGAACTCAGC CCGCTGCTGC 2340
TGTCTACAAC AGAGTGGCAG ATACTGCCCT GCGCCTTCAC CACCCTACCG GCTTTGTCCA 2400
CTGGTTTGAT CCATCTCCAT CAGAACATCG TGGACGTGCA ATACCTGTAC GGTGTAGGGT 2460
CAGCGTTTGT CTCCTTTGCA ATCAAATGGG AGTACATCCT GTTGCTTTTC CTTCTCCTAG 2520
CGGACGCGCG CGTGTGTGCC 2540

Sequence list No.3

Length: 1885

Type: nucleic acid

Strandedness: single

Topology: linear

Molecular Type: cDNA to genomic RNA

```
ACCCGCCCC/ TAATAGGGGC GACACTCCGC CATGAACCAC TCCCCTGTGA GGAACTACTG   60
TCTTCACGCA GAAAGCGTCT AGCCATGGCG TTAGTATGAG TGTCGTACAG CCTCCAGGCC  120
CCCCCCTCCC GGGAGAGCCA TAGTGGTCTG CGGAACCGGT GAGTACACCG GAATTGCCGG  180
GAAGACTGGG TCCTTTCTTG GATAAACCCA CTCTATGCCC GGCCATTTGG GCGCTCCCCC  240
GCAAGACTGC TAGCCGAGTA GCGTTGGGTT GCGAAAGGCC TTGTGGTACT GCCTGATAGG  300
GTGCTTGCGA GTGCCCCGGG AGGTCTCGTA GACCGTGCAC CATGAGCACA AATCCCAAAC  360
CTCAAAGAAA AACCAAAAGA AACACTAACC GTCGCCCACA AGACGTTAAG TTTCCGGGCG  420
GCGGCCAGAT CGTTGGCGGA GTATACTTGT TGCCGCGCAG GGGCCCCAGG TTGGGTGTGC  480
GCGCGACAAG GAAGACTTCG GAGCGGTCCC AGCCACGTGG GAGGCGCCAG CCCATCCCCA  540
AAGATCGGCG CTCCACTGGC AAGTCCTGGG GAAAGCCAGG ATATCCCTGG CCCCTATATG  600
GGAATGAGGG GCTCGGCTGG GCAGGATGGC TCCTGTCCCC CCGAGGTTCC CGTCCCTCTT  660
GGGGCCCCAA TGACCCCCGG CATAGGTCGC GCAATGTGGG TAAGGTCATC GATACCCTAA  720
CGTGCGGCTT TGCCGACCTC ATGGGGTACA TCCCCGTCGT GGGCGCCCCG CTTGGTGGCG  780
TCGCCAGAGC TCTCGCACAC GGCGTGAGAG TCCTGGAGGA CGGGGTTAAC TATGCAACAG  840
GGAACTTACC TGGTTGCTCC TTTTCTATTT TCTTGCTGGC GCTACTGTCC TGCATCACCG  900
TCCCGGTCTC TGCTGTCCAG GTTAAGAACA CCAGTAACAG CTATATGGTG ACTAACGACT  960
GTTCCAATGA CAGCATTACC TGGCAGCTCC AGGGCGCGGT TCTCCACGTC CCCGGGTGCG 1020
TCCCGTGCGA GAAAGTGGGG AATATGTCAC GGTGCTGGAT ACCGGTCTCA CCGAACGTGG 1080
CTGTGCGGCA GCCCGGCGCC CTCACGCAGG GTCTGCGGAC GCACATCGAC ATGGTTGTGG 1140
TGTCCGCTAC GCTCTGCTCC GCTCTCTACG TGGGGGACCT CTGCGGTGGG GTGATGCTCG 1200
```

CGGCCCAGAT GTTCATCGTC TCGCCGCAGC ACCACTGGTT TGTGCGGGAA TGCAATTGCT 1260
CCATCTACCC TGGTGCCATC ACTGGACACC GTATGGCATG GGACATGATG ATGAACTGGT 1320
CACCCACGGC CACCATGATC CTGGCGTACG CGATGCGCGT CCCCGAGGTT ATCATAGACA 1380
TCATTAGCGG GGCTCACTGG GGCGTCATGT TCGGCCTAGC CTACTTCTCT ATGCAGGGAG 1440
CGTGGGCGAA GGTCGTTGTC ATCCTTCTGC TGGCCGCTGG AGTGGATGCG AACACCCGCA 1500
CCGTTGCGGG TTCTGCTGCG GCAACCACCA GGGGCTTCAC CAGCATGTTC TCCTCTGGCT 1560
CGAAGCAGAA CCTTCAGCTC ATTAACACCA ACGGTAGCTG GCACATCAAC CGCACTGCCC 1620
TGAATTGCAA TGACTCCTTG AACACCGGCT TTATCGCGTC CCTGTTCTAC GTCAACCGCT 1680
TCAATTCGTC AGGATGTCCC CATCGCCTGT CCGTCTGCCG CAGCATCGAG GCTTTCCGGA 1740
TAGGGTGGGG CACCTTGCAA TACGAGGATA ATGTCACCAA TCCAGAAGAT ATGAGACCAT 1800
ATTGCTGGCA CTACCCACCA AAACCGTGTG GCATAGTCCC CGCGAGGTCT GTGTGCGGCC 1860
CAGTGTACTG TTTCACCCCC AGCCCA 1886-1=1885

Sequence list No.4

Length: 2551

Type: nucleic acid

Strandedness: single

Topology: linear

Molecular Type: cDNA to genomic RNA

```
ACCCGCCCC/ TAATAGGGGC GACACTCCGC CATGAACCAC TCCCCTGTGA GGAACTACTG   60
TCTTCACGCA GAAAGCGTCT AGCCATGGCG TTAGTATGAG TGTCGTACAG CCTCCAGGCC  120
CCCCCCTCCC GGGAGAGCCA TAGTGGTCTG CGGAACCGGT GAGTACACCG GAATTGCCGG  180
GAAGACTGGG TCCTTTCTTG GATAAACCCA CTCTATGCCC GGTCATTTGG GCGTGCCCCC  240
GCAAGACTGC TAGCCGAGTA GCGTTGGGTT GCGAAAGGCC TTGTGGTACT GCCTGATAGG  300
GTGCTTGCGA GTGCCCCGGG AGGTCTCGTA GACCGTGCAC CATGAGCACA AATCCTAAAC  360
CTCAAAGAAA AACCAAAAGA AACACCAACC GTCGCCCACA AGACGTTAAG TTTCCGGGCG  420
GCGGCCAGAT CGTTGGCGGA GTATACTTGT TGCCGCGCAG GGGCCCCAGG TTGGGTGTGC  480
GCGCGACAAG GAAGACTTCG GAGCGGTCCC AGCCACGTGG AAGGCGCCAG CCCATCCCTA  540
AGGATCGGCG CTCCACTGGC AAATCCTGGG GAAAACCAGG ATACCCCTGG CCCCTATACG  600
GGAATGAGGG ACTCGGCTGG GCAGGATGGC TCCTGTCCCC CCGAGGTTCC CGTCCCTCTT  660
GGGGCCCCAA TGACCCCCGG CATAGGTCCC GCAACGTGGG TAAGGTCATC GATACCCTAA  720
CGTGCGGCTT TGCCGACCTC ATGGGGTACA TCCCTGTCGT AGGCGCCCCG CTCGGCGGCG  780
TCGCCAGAGC TCTCGCGCAT GGCGTGAGAG TCCTGGAGGA CGGGGTTAAT TTTGCAACAG  840
GGAACTTACC CGGTTGCTCC TTTTCTATCT TCTTGCTGGC CCTGCTGTCC TGCATCACCA  900
CCCCGGTCTC CGCTGCCGAA GTGAAGAACA TCAGTACCGG CTACATGGTG ACCAACGACT  960
GCACCAATGA TAGCATTACC TGGCAACTCC AGGCTGCTGT CCTCCACGTC CCCGGGTGCG 1020
TCCCGTGCGA GAAAGTGGGG AATACATCTC GGTGCTGGAT ACCGGTCTCA CCGAATGTGG 1080
CCGTGCAGCA GCCCGGCGCC CTCACGCAGG GCTTACGGAC GCACATTGAC ATGGTTGTGA 1140
TGTCCGCCAC GCTCTGCTCC GCTCTTTACG TGGGGGACCT CTGCGGTGGG GTGATGCTTG 1200
```

CAGCCCAGAT GTTCATTGTC TCGCCACAGC ACCACTGGTT TGTGCAAGAC TGCAATTGCT 1260
CCATCTACCC TGGTACCATC ACTGGACACC GCATGGCGTG GGACATGATG ATGAACTGGT 1320
CGCCCACGGC TACCATGATC CTGGCGTACG CGATGCGCGT CCCCGAGGTC ATCATAGACA 1380
TCATTGGCGG GGCTCATTGG GGCGTCATGT TCGGCTTAGC CTACTTCTCT ATGCAGGGAG 1440
CGTGGGCAAA AGTCGTTGTC ATTCTTTTGC TGGCCGCCGG GGTGGACGCG CAAACCCATA 1500
CCGTTGGGGG TTCTACCGCG CATAACGCCA GGACCCTCAC CGGCATGTTC TCCCTTGGTG 1560
CCAGGCAGAA AATCCAGCTC ATCAACACCA ATGGCAGTTG GCACATCAAC CGCACCGCCC 1620
TGAACTGCAA TGACTCTTTG CACACCGGCT TCCTCGCGTC ACTGTTCTAC ACCCACAGCT 1680
TCAACTCGTC AGGATGTCCC GAACGCATGT CCGCCTGCCG CAGTATCGAG GCCTTTCGGG 1740
TGGGATGGGG CGCCTTACAA TATGAGGACA ATGTCACCAA TCCAGAGGAT ATGAGACCGT 1800
ATTGCTGGCA CTACCCACCA AGACAGTGTG GTGTAGTCTC CGCGAGCTCT GTGTGTGGCC 1860
CAGTGTACTG TTTCACCCCC AGCCCAGTAG TAGTGGGTAC GACCGATAGA CTTGGAGCGC 1920
CCACTTACAC GTGGGGGGAG AATGAGACAG ATGTCTTCCT ATTGAACAGC ACTCGACCAC 1980
CGCAGGGGTC ATGGTTCGGC TGCACGTGGA TGAACTCCAC TGGCTACACC AAGACTTGCG 2040
GCGCACCACC CTGCCGCATT AGAGCTGACT TCAATGCCAG CATGGACTTG TTGTGCCCCA 2100
CGGACTGTTT TAGGAAGCAT CCTGATACCA CCTACATCAA ATGTGGCTCT GGGCCCTGGC 2160
TCACGCCAAG GTGCCTGATC GACTACCCCT ACAGGCTCTG GCATTACCCC TGCACAGTTA 2220
ACTATACCAT CTTCAAAATA AGGATGTATG TGGGGGGGGT CGAGCACAGG CTCACGGCTG 2280
CGTGCAATTT CACTCGTGGG GATCGTTGCA ACTTGGAGGA CAGAGACAGA AGTCAACTGT 2340
CTCCTTTGCT GCACTCCACC ACGGAGTGGG CCATTTTACC TTGCACTTAC TCGGACCTGC 2400
CCGCCTTGTC GACTGGTCTT CTCCACCTCC ACCAAAACAT CGTGGACGTG CAATTCATGT 2460
ATGGCCTATC ACCTGCTCTC ACAAAATACA TCGTCCGATG GGAGTGGGTA GTACTCTTAT 2520
TCCTGCTCTT AGCGGACGCC AGGGTTTGCG CC 2552-1=2551

Sequence list No.5

Length: 2498

Type: nucleic acid

Strandedness: single

Topology: linear

Molecular Type: cDNA to genomic RNA

```
GCCCGCCCCC TGATGGGGGC GACACTCCGC CATGAATCAC TCCCCTGTGA GGAACTACTG   60
TCTTCACGCA GAAAGCGTCT AGCCATGGCG TTAGTATGAG TGTCGTACAG CCTCCAGGCC  120
CCCCCCTCCC GGGAGAGCCA TAGTGGTCTG CGGAACCGGT GAGTACACCG GAATTGCCGG  180
AAAGACTGGG TCCTTTCTTG GATCAACCCA CTCTATGTCC GGTCATTTGG GCGTGCCCCC  240
GCAAGACTGC TAGCCTAGTA GCGTTGGGTT GCGAACGGCC TTGTGGTACT GCCTGATAGG  300
GTGCTTGCGA GTGCCCCGGG AGGTCTCGTA GACCGTGCAT TATGAGCACA AATCCTAAAC  360
CTCAAAGAAA AACCAAAAGA AACACAAACC GCCGCCCACA GGACGTCAAG TTCCCGGGTG  420
GCGGCCAGAT CGTTGGCGGA GTTTACTTGC TGCCGCGCAG GGGCCCCAGG TTGGGTGTGC  480
GCGCGACAAG GAAGACTTCC GAGCGATCCC AGCCGCGTGG GAGACGCCAG CCCATCCCGA  540
AAGATCGGCG CTCCACCGGC AAGTCCTGGG GAAAGCCAGG ATATCCTTGG CCTCTGTATG  600
GAAACGAGGG CTGCGGCTGG GCAGGTTGGC TCCTGTCCCC CCGCGGGTCT CGTCCTACTT  660
GGGGCCCCAC CGACCCCCGG CATAGATCAC GCAATTTGGG CAAGGTCATC GATACCCTTA  720
CGTGTGGTTT TGCCGACCTC ATGGGGTACA TCCCTGTCGT TGGCGCTCCT GTTGGAGGCG  780
TCGCCAGAGC TCTGGCACAC GGTGTTAGGG TCCTGGAAGA CGGGATAAAT TATGCAACAG  840
GGAATCTGCC TGGTTGCTCT TTTTCTATCT TCTTACTTGC TCTTCTGTCG TGCGTCACAG  900
TGCCAGTGTC TGCAGTGGAA GTCAGGAACA TCAGTTCCAG CTACTATGCC ACCAATGATT  960
GCTCGAACAA CAGCATCACC TGGCAGCTCA CTAACGCAGT TCTCCACCTT CCCGGATGCG 1020
TCCCATGTGA GAATGACAAC GGCACCTTGC GCTGCTGGAT ACAAGTAACA CCTAATGTGG 1080
CTGTGAAACA TCGCGGTGCA CTCACCCACA ACCTGCGAAC ACATGTCGAC ATGATCGTGA 1140
TGGCAGCTAC GGTCTGCTCG GCCTTGTATG TGGGAGACAT ATGTGGGGCC GTGATGATTG 1200
```

CGTCGCAGGC TTTCATAATA TCGCCAGAAC GCCATAACTT CACCCAAGAG TGCAACTGTT 1260
CCATCTACCA AGGCCATATC ACTGGTCACC GCATGGCATG GGACATGATG TTAAACTGGT 1320
CACCAACTCT TACCATGATC CTCGCCTATG CCGCTCGTGT TCCTGAGCTA GTCCTTGAGG 1380
TTGTCTTCGG CGGCCATTGG GGCGTGGTGT TTGGCTTGGC CTATTTCTCC ATGCAGGGAG 1440
CGTGGGCCAA AGTCATTGCC ATCCTCCTCC TTGTCGCAGG AGTGGATGCG AGCACCCAAG 1500
TCACTGGCGG ACAAGCGGCC CATACCGTTA GAGGAGTCGC CAGCATCTTC AGCCCTGGCT 1560
CCCGGCAGGA TATCAGTCTA ATCAACACCA ATGGCAGCTG GCACATAAAC CGGACCGCCC 1620
TCAATTGCAA TGATAGCTTG CAAACAGGTT TCTTCGCTGC CCTGTTTTAC GTCAGACGTT 1680
TCAACAGCTC TGGCTGCCCC GAGCGCTTGT CTTCCTGCCG TAAGCTGGAT GATTTTCGCA 1740
TCGGGTGGGG AACCTTGGAA TATGAGACCA ATGTTACCAA CGAGGAGGAC ATGAGACCGT 1800
ACTGCTGGCA TTACCCTCCG AAGCCTTGCG GCATCGTCTC GGCTAAGACG GTTTGCGGAC 1860
CGGTCTATTG TTTCACCCCT AGCCCTGTTG TCGTGGGCAC CACTGACAAG CAGGGCGTAC 1920
CCACCTACAC CTGGGGAGAA AACGAGACCG ATGTCTTCCT GCTGAATAGC ACAAGACCCC 1980
CGCGAGGAGC TTGGTTCGGC TGCACCTGGA TGAACGGGAC TGGGTTCACT AAGACATGCG 2040
GTGCACCACC TTGCCGCATT AGGAAAGACT ACAACAGCAC TATCGATTTA TTGTGCCCCA 2100
CAGACTGTTT TAGGAAGCAC CCAGATGCTA CCTATCTTAA GTGTGGAGCA GGGCCTTGGT 2160
TAACTCCCAG GTGCCTGGTA GACTACCCTT ATAGGTTGTG GCATTATCCG TGCACTGTAA 2220
ACTTCACCAT CTTCAAGGCG CGGATGTATG TAGGAGGGGT GGAGCATCGA TTCTCCGCAG 2280
CATGCAACTT CACGCGCGGA GATCGCTGCA GACTGGAAGA TAGGGATAGG GGCCAGCAGA 2340
GTCCACTGCT GCATTCCACT ACTGAGTGGG CGGTGCTCCC ATGCTCCTTC TCTGACCTAC 2400
CAGCACTATC CACTGGCCTA TTGCACCTCC ACCAAAACAT CGTGGACGTG CAGTACCTCT 2460
ACGGACTTTC TCCGGCTCTG ACAAGATACA TCGTGAAG 2498

Sequence list No.6

Length: 1173

Type: nucleic acid

Strandedness: single

Topology: linear

Molecular Type: cDNA to genomic RNA

```
CACAGTCACT GAGAGCGATA TCCGTACGGA GGAGGCAATC TACCAGTGTT GTGACCTGGA   60
CCCCCAAGCC CGCGTGGCCA TCAAGTCCCT CACCGAGAGG CTTTATGTCG GGGGCCCTCT  120
TACCAATTCA AGGGGGGAAA ACTGCGGCTA TCGCAGGTGC CGCGCCAGCG GCGTACTGAC  180
AACTAGCTGT GGTAACACCC TCACTTGCTA CATCAAGGCC CAAGCAGCCT GTCGAGCCGC  240
AGGGCTCCGG GACTGCACCA TGCTCGTGTG TGGCGACGAC CTAGTCGTTA TCTGTGAAAG  300
TCAGGGAGTC CAGGAGGATG CAGCGAGCCT GAGAGCCTTC ACGGAGGCTA TGACCAGGTA  360
CTCCGCTCCC CCCGGGGACC CCCCCCAACC AGAATACGAC TTGGAGCTCA TAACATCGTG  420
CTCCTCTAAC GTGTCAGTCG CCCACGATGG CACTGGAAAG AGGGTCTATT ACCTTACCCG  480
TGACCCTACA ACTCCCCTCG CGAGAGCCGC GTGGGAGACA GCAAGGCACA CTCCAGTCAA  540
TTCCTGGCTA GGCAACATAA TCATGTTTGC TCCTACATTG TGGGCGAGGA TGATACTGAT  600
GACCCACTTC TTCAGTGTCC TCATAGCCAG GGATCAGCTT GAACAGGCCC TTGATTGCGA  660
AATCTACGGA GCCTGCTACT CCATAGAACC ACTGGATCTA CCTCCAATTA TTCAAAGACT  720
CCATGGCCTC AGCGCGTTTT CACTCCACAG TTACTCTCCA GGTGAAATCA ATAGGGTGGC  780
CGCATGCCTC AGAAAACTTG GGGTTCCGCC CTTGCGAGCT TGGAGACACC GGGCCCGGAG  840
CGTCCGCGCT AGACTTCTGT CCAGAGGAGG CAGGGCTGCC ATATGTGGC AAGTACCTCTT  900
CAACTGGGCA GTAAGAACAA AGCTCAAACT CACTCCAATA GCGGCCGCTG GCCGGCTGGA  960
CTTGTCCGGC TGGTTCACGG CTGGCTACAG CGGGGGAGAC ATTTATCACA GCGTGTCTCA 1020
TGCCCGGCCC CGCTGGTTCT GGTTCTGCCT ACTCCTGCTT GCTGCAGGGG TAGGCATCTA 1080
CCTCCTCCCC AACCGATAAA GGTTGGGGTA AACACTCCGG CCTCTTAGGC CATTTTCTGT 1140
GTTTTTTTTT TTTTTTTTTT TTTTTTTTTT TTT  1173
```

Sequence list No.7

Length: 1173

Type: nucleic acid

Strandedness: single

Topology: linear

Molecular Type: cDNA to genomic RNA

```
AACGGTCACT GAGAGTGACA TTCGTGTTGA GGAGTCAATT TACCAATGTT GTGACTTGGC   60
CCCCGAGGCC AGACAGGCCA TAAGGTCGCT CACGGAGCGG CTTTACATCG GGGGTCCCCT  120
GACTAATTCA AAAGGGCAGA ACTGCGGTTA TCGCCGGTGC CGCGCAAGTG GCGTGCTGAC  180
GACTAGCTGC GGTAATACCC TCACATGTTA CTTGAAGGCC ACTGCGGCCT GTCGAGCTGC  240
AAAGCTCCAG GACTGCACGA TGCTCGTGAA ACGGAGAGAC CTTGTCGTTA TCTGTGAAAG  300
CGCGGGAACC CAGGAGGATG CGGCGGCCCT ACGAGTCTTC ACGGAGGCTA TGACTAGGTA  360
CTCCGCCCCC CCCGGGGATC CGCCCCAACC AGAGTACGAC CTGGAGCTGA TAACATCATG  420
TTCCTCCAAC GTGTCAGTCG CGCACGATGC ATCTGGCAAA AGGGTATACT ACCTCACCCG  480
TGACCCCACC ATCCCCCTTG CACGGGCTGC GTGGGAGACA GCTAGACACA CTCCAGTCAA  540
CTCTTGGCTA GGCAATATCA TCATGTATGC GCCCGCCCTA TGGGCAAGGA TGATTCTGAT  600
GACTCACTTT TTCTCCATCC TTCTAGCTCA AGAGCAACTT GAACAAGCCC TGGATTGTCA  660
GATCTACGGG GCCTGCTACT CCATTGAGCC ACTTGACCTA CCTCAGATCA TTGAACGACT  720
CCATGGTCTT AGCGCATTTT CACTCCACAG TTACTCTCCA GGTGAGATCA ATAGGGTGGC  780
TTCATGCCTC AGGAAACTTG GGGTACCACC CTTGCGAGTC TGGAGACATC GGGCCAGAAG  840
TGTCCGCGCT AAGCTACTGT CCCAGGGGGG GAGGGCCGCC ACTTGTGGCA GATACCTCTT  900
TAACTGGGCA GTAAGGACCA AGCTTAAACT CACTCCAATC CCGGCTGCGT CCCAGCTGGA  960
CTTGTCCGGC TGGTTCGTCG CTGGTTACAG CGGGGGAGAC ATATATCACA GCCTGTCTCG 1020
TGCCCGACCC CGCTGGTTTC TGTTGTGCCT ACTCCTACTT TCTGTAGGGG TAGGCATTTA 1080
CCTGCTCCCC AACCGATGAA CGGGGAGCTA ACCACTCCAG GCCAATAGGC CATCCCGTTT 1140
TTTTTTTTTT TTTTTTTTTT TTTTTTTTTT TTT   1173
```

Sequence list No.8

Length: 1173
Type: nucleic acid
Strandedness: single
Topology: linear
Molecular Type: cDNA to genomic RNA

GACACCCGCT GTTTTGACTC AACCGTTACT GAGAGAGACA TTAGAACTGA GGAGTCCATA 60
TACCAGGCCT GCTCCCTGCC TGAGGAGGCC CGCACTGCTA TACACTCGCT GACTGAGAGA 120
CTCTACGTGG GAGGGCCCAT GTTCAACAGC AAGGGCCAGA CCTGCGGGTA CAGGCGTTGC 180
CGTGCCAGCG GGGTGCTCAC CACTAGCATG GGGAACACCA TCACATGCTA TGTGAAAGCC 240
CTAGCGGCTT GCAAGGCTGC GGGGATAGTA GCGCCCACAA TGCTGGTATG TGGCGACGAC 300
TTGGTCGTCA TCTCAGAAAG CCAGGGGACT GAGGAGGACG AGCGGAACCT GAGAGCCTTC 360
ACGGAGGCCA TGACCAGGTA TTCTGCCCCT CCCGGTGACC CCCCCAGACC GGAATACGAC 420
CTGGAGCTGA TAACATCTTG TTCCTCAAAT GTGTCTGTGG CGCTGGGCCC ACAGGGCCGC 480
CGCAGATACT ACCTGACCAG AGACCCTACC ACTCCATTCG CCCGGGCTGC CTGGGAAACA 540
GTTATACACT CCCCTGTCAA TTCATGGCTG GGAAACATCA TCCAGTACGC TCCAACCATC 600
TGGGTTCGCA TGGTCCTGAT GACACATTTT TTTTCCATCC TCATGGCCCA AGACACCCTG 660
GACCAGAACC TCAACTTTGA GATGTACGGA GCGGTGTACT CCGTGAGTCC TCTGGACCTC 720
CCAGCCATAA TTGAAAGGCT ACACGGGCTT GACGCCTTTT CTCTGCATAC ATACACTCCC 780
AACGAACTGA CGCGGGTGGC TTCAGCCCTC AGAAAACTTG GGGCGCCACC CCTCAGAACG 840
TGGAAGAGTC GGGCGCGTGC AGTAAGGGCG TCCCTCATCT CCCGTGGAGG GAGAGCGGCC 900
GTTTGCGGTC GGTATCTCTT CAACTGGGCG GTGAAGACCA AGCTCAAACT CACTCCATTG 960
CCGGAGGCGC GCCTCCTGGA TTTATCCAGT TGGTTCACCG TCGGCGCCGG CGGGGGCGAC 1020
ATTTATCACA GCGTGTCGCG TGCCCGACCC CGCCTATTAC TCCTTAGCCT ACTCCTGCCT 1080
TCTGTAGGAG TAGGCCTCTT TTTACTCCCC GCTCGGTAGA GCGGCACACA TTAGCTACAC 1140
TCCATAGCTA ACTGTTCCTT TTTTTTTTTT TTT 1173

Sequence list No.9

Length: 1173
Type: nucleic acid
Strandedness: single
Topology: linear
Molecular Type: cDNA to genomic RNA

```
AACCGTCACT GAGAGAGACA TCAGGACTGA GGAGTCCATA TATCGGGCCT GTTCCTTGCC   60
CGAGGAGGCC CACACTGCCA TACACTCGCT GACTGAGAGA CTTTACGTGG GAGGGCCCAT  120
GTTCAACAGC AAGGGCCAAA CCTGCGGGTA CAGGCGTTGC CGCGCCAGCG GGGTGCTCAC  180
CACTAGCATG GGGAACACCA TCACATGCTA CGTGAAAGCC TTAGCGGCCT GTAAGGCTGC  240
AGGGATAATT GCGCCCACAA TGCTGGTATG CGGCGATGAC TTGGTTGTCA TCTCAGAAAG  300
CCAGGGGACC GAGGAGGACG AGCGGAACCT GAGAGCCTTC ACGGAGGCTA TGACCAGGTA  360
TTCTGCCCCT CCTGGTGACC CCCCCAGACC GGAATATGAC CTGGAGCTGA TAACATCTTG  420
CTCCTCAAAT GTGTCTGTGG CGTTGGGCCC ACAAGGCCGC CGCAGATACT ACCTGACCAG  480
AGACCCTACC ACTCCAATCG CCCGGGCTGC CTGGGAAACA GTTAGACACT CCCCTGTCAA  540
TTCATGGCTA GGAAACATCA TCCAGTACGC CCCAACCATA TGGGCTCGCA TGGTCCTGAT  600
GACACACTTC TTCTCCATTC TCATGGCCCA AGATACTCTG GACCAGAACC TCAACTTTGA  660
GATGTACGGA GCGGTGTACT CCGTGAGTCC CTTGGACCTC CCAGCCATAA TTGAAAGGTT  720
ACACGGGCTT GACGCTTTCT CTCTGCACAC ATACACTCCC CACGAACTGA CACGGGTGGC  780
TTCAGCCCTC AGAAAACTTG GGGCGCCACC CCTCAGAGCG TGGAAGAGCC GGGCACGTGC  840
AGTCAGGGCG TCCCTCATCT CCCGTGGGGG GAGAGCGGCC GTTTGCGGCC GATATCTCTT  900
CAACTGGGCG GTGAAGACCA AGCTCAAACT CACTCCATTG CCGGAAGCGC GCCTCCTGGA  960
TTTATCCAGC TGGTTCACTG TCGGCGCCGG CGGGGGCGAC ATTTATCACA GCGTGTCGCG 1020
TGCCCGACCC CGCTTATTAC TCCTTGGCCT ACTCCTACTT TTTGTAGGGG TAGGCCTTTT 1080
CCTACTCCCC GCTCGGTAGA GCGGCACACA TTAGCTACAC TCCATAGCTA ACTGTCCCTT 1140
TTTTTTTTTT TTTTTTTTTT TTTTTTTTTT TTT  1173
```

Sequence list No.10

Length: 1173

Type: nucleic acid

Strandedness: single

Topology: linear

Molecular Type: cDNA to genomic RNA

```
CACCGTCACG GAGAGGGACA TAAGAACAGA AGAATCCATA TATCAGGCTT GTTCCCTGCC   60
TCAGGAGGCC AGAACTGCCA TACACTCGCT CACTGAGAGA CTCTACGTAG GAGGGCCCAT  120
GACAAACAGC AAAGGCCAAT CCTGCGGCTA CAGGCGTTGC CGCGCAAGCG GGGTTTTCAC  180
CACCAGCATA GGGAATACCA CGACATGCTA CATCAAAGCA CTTGCGGCGT GCAAAGCTGC  240
AGGGATCAAG GACCCTATCA TGCTGGTGTG TGGAGACGAC CTGGTCGTCA TCTCAGAGAG  300
CCAAGGTAAC GAGGAGGACG AGCGAAACCT GAGAGCTTTC ACGGAGGCTA TGACCAGGTA  360
TTCAGCCCCT CCCGGTGACC TTCCCAGACC GGAATATGAC TTGGAGCTTA TAACATCCTG  420
CTCCTCAAAC GTATCGGTAG CGCTGGACCC TCGGGGTCGC CGCCGGTACT ACCTAACCAG  480
AGACCCTACC ACTCCAATCT CCCGAGCTGC TTGGGAAACA GTAAGACACT CCCCCGTCAA  540
TTCTTGGCTG GGCAACATCA TCCAATACGC CCCCACGATC TGGGTCCGGA TGGTCATAAT  600
GACCCACTTT TTCAACATAC TGCTGGCCCA GGACACTCTG AACCAAAATC TCAATTTTGA  660
GATGTACGGG GCAGTATATT CGGTCAATCC ATTAGACCTA CCAGCCATAA TTGAAAGGCT  720
ACATGGGCTT GATGCCTTTT CACTGCACAC ATACTCTCCC CACGAACTCT CGCGGGTGGC  780
AGCGACTCTC AGAAAACTTG GAGCGCCTCC CCTTAGAGCG TGGAAGAGTC GGGCGCGTGC  840
CGTGAGGGCC TCACTCATCG CCCAGGGACC GAGAGCGGCC ATCTGTGGCC GTTACCTCTT  900
CAACTGGGCG GTGAAGACAA AGCTCAAACT CACTCCATTG CCCGAGGCGG CCCGCCTGGA  960
TTTATCCGGG TGGTTCACCG TGGGCGCCGG CGGGGGCGAC ATCTTTCACA GCGTGTCGCA 1020
TGCCCGACCC CGCCTATTAC TCCTTTGCCT ACTCCTACTT AGCGTAGGAG TAGGCATCTT 1080
TTTACTCCCC GCTCGGTAGA GCGGCAAACC CTAGCTACAC TCCATAGCTA GTTCCTTTTT 1140
TTTTTTTTTT TTTTTTTTTT TTTTTTTTTT TTT 1173
```

Sequence list No.11

Length: 20

Type: nucleic acid

Strandedness: single

Topology: linear

Molecular Type: cDNA to genomic RNA (#23)

TAGATTGGGT GTGCGCGCGA 20

Sequence list No.12

Length: 20

Type: nucleic acid

Strandedness: single

Topology: linear

Molecular Type: cDNA to genomic RNA (#25)

TCCCTGTTGC ATAGTTCACG 20

Sequence list No.13

Length: 20
Type: nucleic acid
Strandedness: single
Topology: linear
Molecular Type: cDNA to genomic RNA
(#32)

ACTCCACCAT AGATCACTCC 20

Sequence list No.14

Length: 20
Type: nucleic acid
Strandedness: single
Topology: linear
Molecular Type: cDNA to genomic RNA
(#33)

TTCACGCAGA AAGCGTCTAG 20

Sequence list No.15

Length: 20
Type: nucleic acid
Strandedness: single
Topology: linear
Molecular Type: cDNA to genomic RNA
(#36)

AACACTACTC GGCTAGCAGT 20

Sequence list No.16

Length: 20
Type: nucleic acid
Strandedness: single
Topology: linear
Molecular Type: cDNA to genomic RNA
(#38)

TGCAATTGTT CTATCTACCC 20

Sequence list No.17

Length: 20
Type: nucleic acid
Strandedness: single
Topology: linear
Molecular Type: cDNA to genomic RNA
(#41)

CAGGGCTTGG GGTGAAGCAA 20

Sequence list No.18

Length: 20
Type: nucleic acid
Strandedness: single
Topology: linear
Molecular Type: cDNA to genomic RNA
(#48)

GTTGATCCAA GAAAGGACCC 20

Sequence list No.19

Length: 20

Type: nucleic acid

Strandedness: single

Topology: linear

Molecular Type: cDNA to genomic RNA

(#50)

GCCGATCTCA TGGGGTACAT 20

Sequence list No.20

Length: 20

Type: nucleic acid

Strandedness: single

Topology: linear

Molecular Type: cDNA to genomic RNA

(#51)

GACCGCTCCG AAGTCTTCCT 20

Sequence list No.21

Length: 20
Type: nucleic acid
Strandedness: single
Topology: linear
Molecular Type: cDNA to genomic RNA
(#54)

ATCGCGTACG CCAGGATCAT 20

Sequence list No.22

Length: 20
Type: nucleic acid
Strandedness: single
Topology: linear
Molecular Type: cDNA to genomic RNA
(#55)

ATTTTCTTGC TGGCCCTGCT 20

Sequence list No.23

Length: 20

Type: nucleic acid

Strandedness: single

Topology: linear

Molecular Type: cDNA to genomic RNA

(#57)

TATTGCTTCA CCCCAAGCCC 20

Sequence list No.24

Length: 20

Type: nucleic acid

Strandedness: single

Topology: linear

Molecular Type: cDNA to genomic RNA

(#61)

TTGCGAGTCT GGAGACATCG 20

Sequence list No.25

Length: 20
Type: nucleic acid
Strandedness: single
Topology: linear
Molecular Type: cDNA to genomic RNA
(#63)

CTGGTGTTCT TAACCTGGAC 20

Sequence list No.26

Length: 20
Type: nucleic acid
Strandedness: single
Topology: linear
Molecular Type: cDNA to genomic RNA
(#78)

TGTCCGCGCT AAGCTACTGT 20

Sequence list No.27

Length: 20

Type: nucleic acid

Strandedness: single

Topology: linear

Molecular Type: cDNA to genomic RNA

(#80)

GACACCCGCT GTTTTGACTC 20

Sequence list No.28

Length: 20

Type: nucleic acid

Strandedness: single

Topology: linear

Molecular Type: cDNA to genomic RNA

(#90)

GCCGTTTGCG GCCGATATCT 20

Sequence list No.29

Length: 20
Type: nucleic acid
Strandedness: single
Topology: linear
Molecular Type: cDNA to genomic RNA
(#91)

GCCATATGTG GCAAGTACCT 20

Sequence list No.30

Length: 20
Type: nucleic acid
Strandedness: single
Topology: linear
Molecular Type: cDNA to genomic RNA
(#97)

AGTCAGGGCG TCCCTCATCT 20

Sequence list No.31

Length: 20
Type: nucleic acid
Strandedness: single
Topology: linear
Molecular Type: cDNA to genomic RNA
(#100)

AAGGCTGCCA TATGTGGCAA 20

Sequence list No.32

Length: 20
Type: nucleic acid
Strandedness: single
Topology: linear
Molecular Type: cDNA to genomic RNA
(#122)

AGGTTCCCTG TTGCATAATT 20

Sequence list No.33

Length: 20
Type: nucleic acid
Strandedness: single
Topology: linear
Molecular Type: cDNA to genomic RNA
(#123)

CTTAGAGCGT GGAAGAGTCG 20

Sequence list No.34

Length: 20
Type: nucleic acid
Strandedness: single
Topology: linear
Molecular Type: cDNA to genomic RNA
(#124)

GCCATCTGTG GCCGTTACCT 20

Sequence list No.35

Length: 20
Type: nucleic acid
Strandedness: single
Topology: linear
Molecular Type: cDNA to genomic RNA
(#127)

ATGTGCCAAC TGCCGTTGGT 20

Sequence list No.36

Length: 20
Type: nucleic acid
Strandedness: single
Topology: linear
Molecular Type: cDNA to genomic RNA
(#128)

TCGGTCGTGC CCACTACCAC 20

Sequence list No.37

Length: 20
Type: nucleic acid
Strandedness: single
Topology: linear
Molecular Type: cDNA to genomic RNA
(#129)

CGCATGGCAT GGGATATGAT 20

Sequence list No.38

Length: 20
Type: nucleic acid
Strandedness: single
Topology: linear
Molecular Type: cDNA to genomic RNA
(#136)

ACTGGGCTGG GAGTGAAACA 20

Sequence list No.39

Length: 20

Type: nucleic acid

Strandedness: single

Topology: linear

Molecular Type: cDNA to genomic RNA

(#145)

CAGGATATCA GTCTAATCAA 20

Sequence list No.40

Length: 20

Type: nucleic acid

Strandedness: single

Topology: linear

Molecular Type: cDNA to genomic RNA

(#148)

TGCACCTGGA TGAACTCAAC 20

Sequence list No.41

Length: 20

Type: nucleic acid

Strandedness: single

Topology: linear

Molecular Type: cDNA to genomic RNA

(#149)

TCTGTGTGTG GCCCAGTGTA 20

Sequence list No.42

Length: 20

Type: nucleic acid

Strandedness: single

Topology: linear

Molecular Type: cDNA to genomic RNA

(#150)

ATCGTCTCGG CTAAGACGGT 20

## Claims

1. An oligonucleotide primer selected from the group consisting of primers #38, #41, #50, #51, #54, #55, #57, #61, #63, #78, #80, #90, #91, #97, #100, #122, #123, #124, #127, #128, #129, #136, #145, #148, #149, and #150. as defined by sequence Listings given hereinbefore.

2. A method of detecting non-A, non-B hepatitis virus comprising synthesizing cDNA from viral RNA and amplifying said cDNA by PCR using at least one oligonucleotide primer according to claim 1.

3. The method according to claim 2, where said primers consist of #38 and #41.

4. The method according to claim 2, where said primers consist of #50 and #54.

5. The method according to claim 2, where said primers consist of #50 and #63.

6. The method according to claim 2, where said primers consist of #50 and #128.

7. The method according to claim 2, where said primers consist of #54 and #55.

8. The method according to claim 2, where said primers consist of #127 and #129.

9. The method according to claim 2, where said primers consist of #136 and #145.

10. A method of detecting non-A, non-B hepatitis virus comprising:
    (1) synthesizing cDNA from viral RNA;
    (2) amplifying said cDNA by PCR in a first stage to produce a product;
    (3) amplifying said product by PCR in a second stage; said amplifying being carried out by using at least one oligonucleotide primer according to claim 1.

11. The method according to claim 10, wherein said primer in step (1) is a pair of primers and said primer in step (2) is a pair of primers, wherein said pair of primers in steps 1 and 2 are selected from the group of pairs of primers consisting of #38 and #41, #50 and #54, #50 and #63, #50 and #128, #54 and #55, #127 and #129, and #136 and #145.

12. A test kit for diagnosing non-A, non-B hepatitis or for detecting non-A, non-B hepatitis virus, said kit comprising:
    (1) at least one primer according to claim 1;
    (2) dATP, dTTP, dGTP, and dCTP; and
    (3) heat stable DNA polymerase.

13. A NANB hepatitis virus detection system comprising at least one oligonucleotide primer according to claim 1 which is used in amplification by PCR of cDNA derived from viral RNA.

Fig. 1

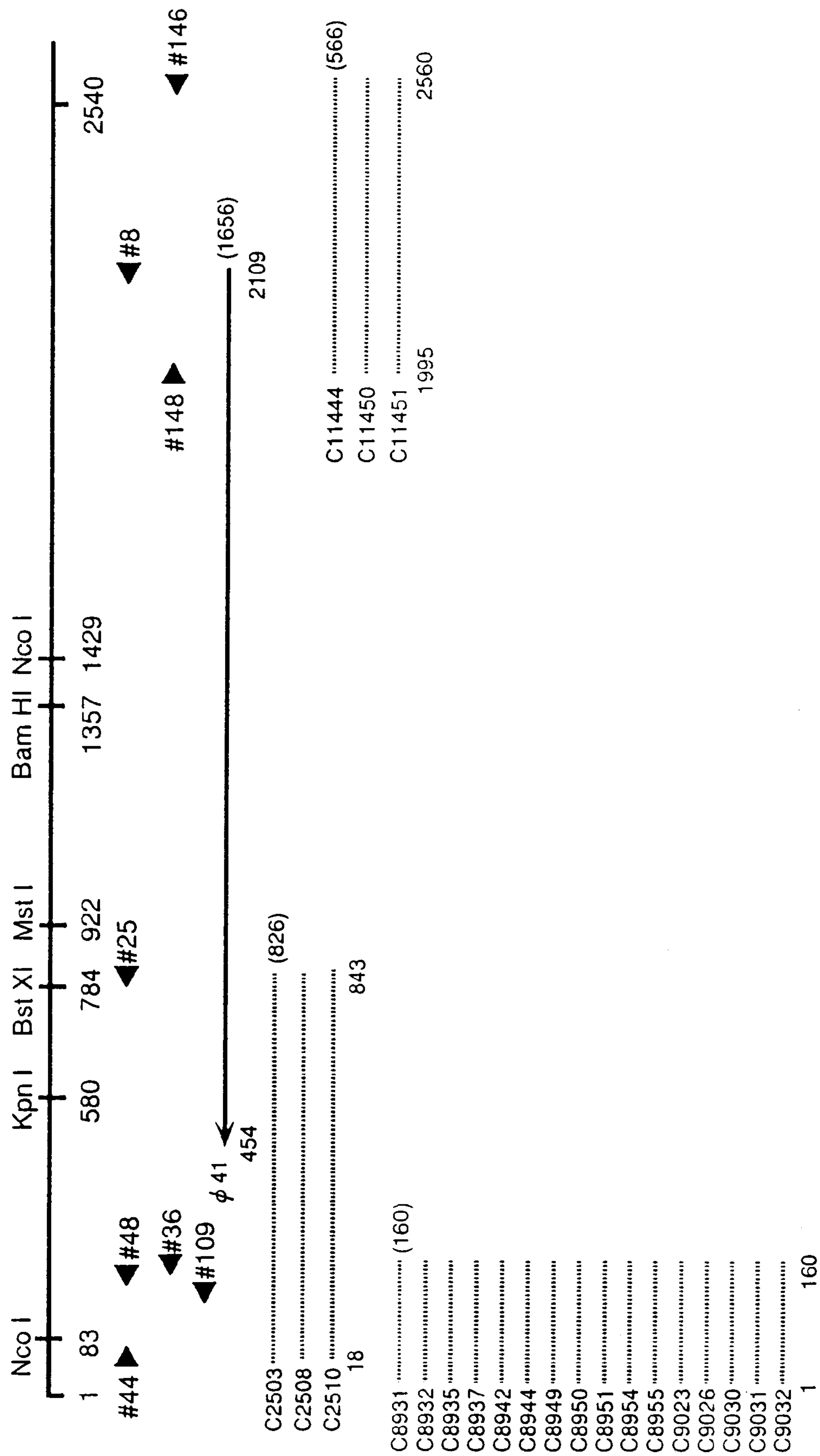

Nco I
Kpn I
Bst XI
Mst I
Bam HI
Nco I
1
83
580
784
922
1357
1429
2540
#44
#48
#36
#109
#25
#8
#148
#146
φ41
454
(1656)
2109
C2503
C2508
C2510
18
(826)
843
C11444
C11450
C11451
1995
(566)
2560
C8931
C8932
C8935
C8937
C8942
C8944
C8949
C8950
C8951
C8954
C8955
C9023
C9026
C9030
C9031
C9032
1
(160)
160

Fig. 2

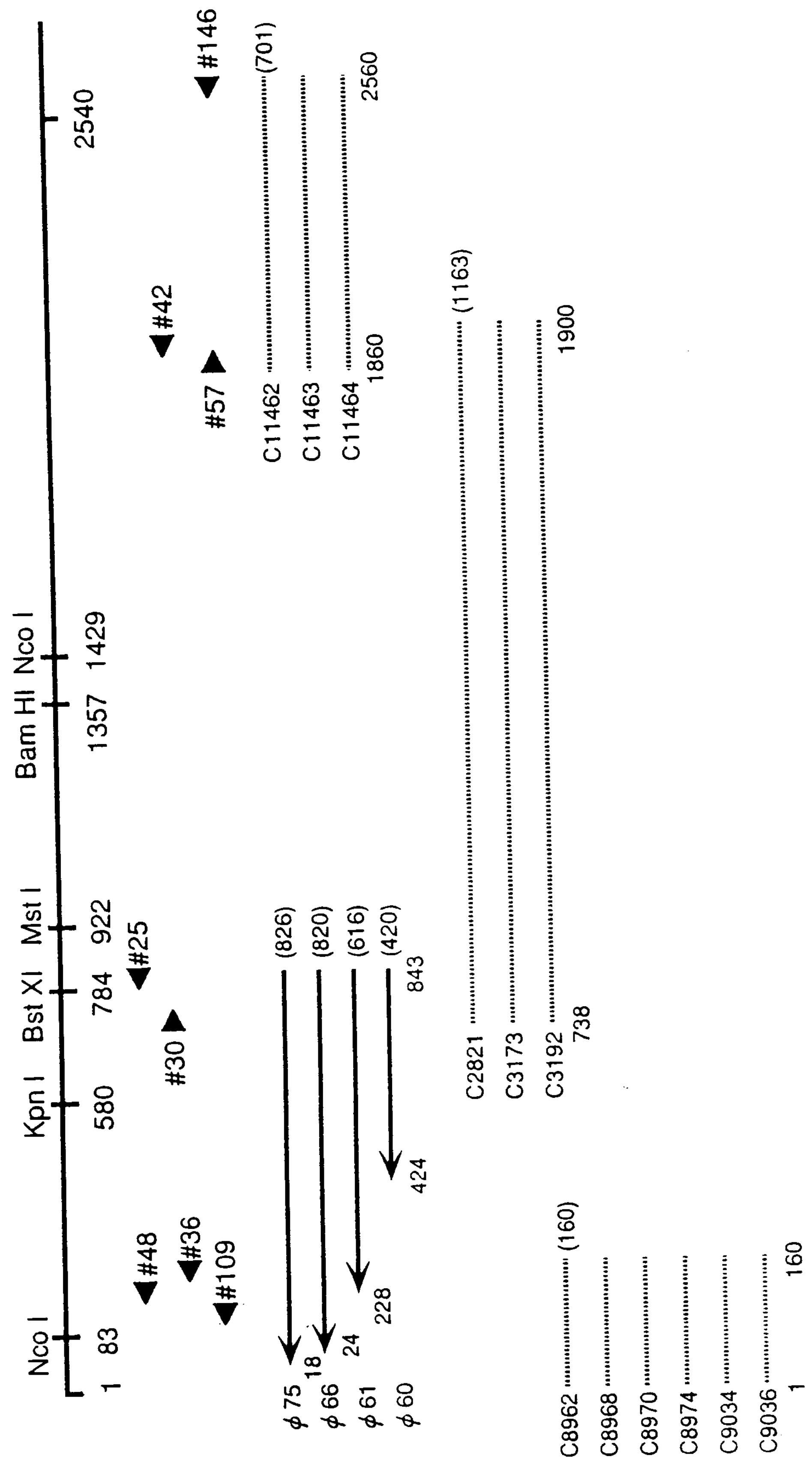
Nco I
Kpn I
Bst XI
Mst I
Bam HI
Nco I
1
83
580
784
922
1357
1429
2540
#48
#36
#109
#30
#25
#42
#57
#146
φ75
φ66
φ61
φ60
18
24
228
424
843
(826)
(820)
(616)
(420)
C11462
C11463
C11464
(701)
1860
2560
C2821
C3173
C3192
(1163)
738
1900
C8962
C8968
C8970
C8974
C9034
C9036
(160)
1
160

Fig. 3

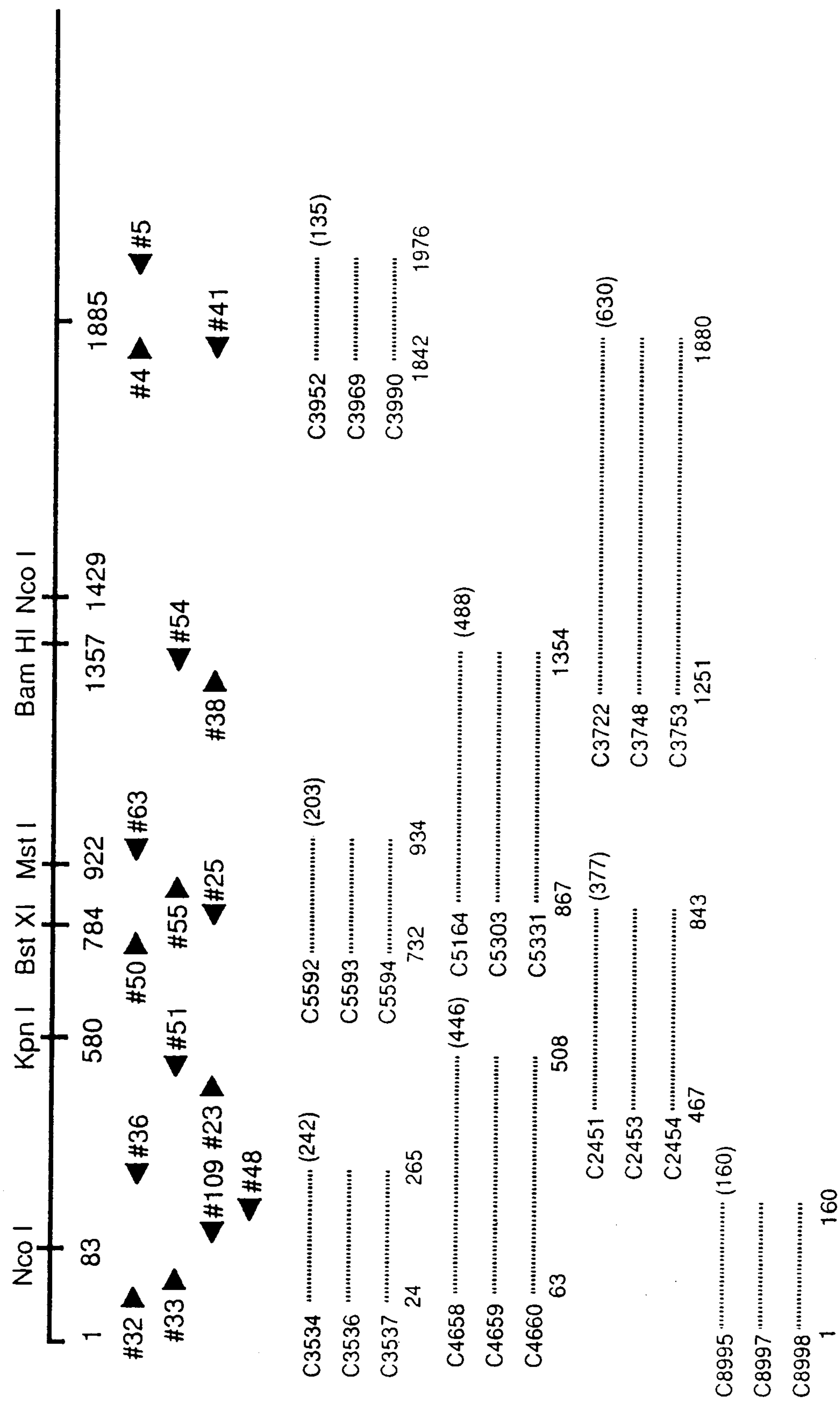
Nco I
Kpn I
Bst XI
Mst I
Bam HI
Nco I
1
83
580
784
922
1357
1429
1885
#32
#33
#36
#109
#23
#48
#51
#50
#55
#25
#63
#38
#54
#4
#41
#5
C3534 (242)
C3536
C3537
24
265
C4658 (446)
C4659
C4660
63
508
C5592 (203)
C5593
C5594
732
934
C5164 (488)
C5303
C5331
867
1354
C2451 (377)
C2453
C2454
467
843
C3722 (630)
C3748
C3753
1251
1880
C3952 (135)
C3969
C3990
1842
1976
C8995 (160)
C8997
C8998
1
160

Fig. 4

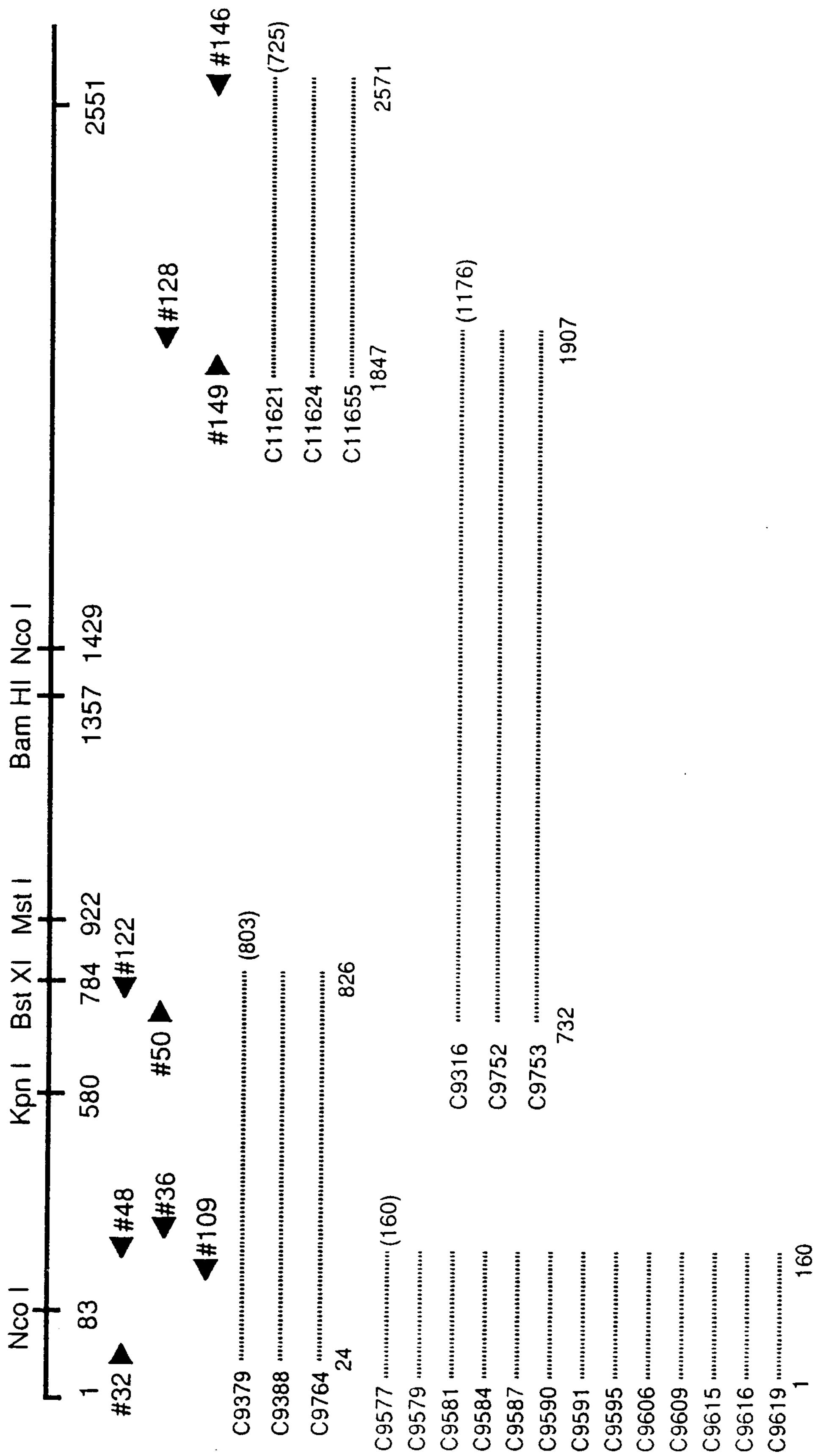

Nco I
Kpn I
Bst XI
Mst I
Bam HI
Nco I
1
83
580
784
922
1357
1429
2551
#32
#48
#36
#109
#50
#122
#128
#149
#146
C9379
C9388
C9764
24
(803)
826
C9577
C9579
C9581
C9584
C9587
C9590
C9591
C9595
C9606
C9609
C9615
C9616
C9619
1
(160)
160
C9316
C9752
C9753
732
(1176)
1907
C11621
C11624
C11655
1847
(725)
2571

Fig. 5

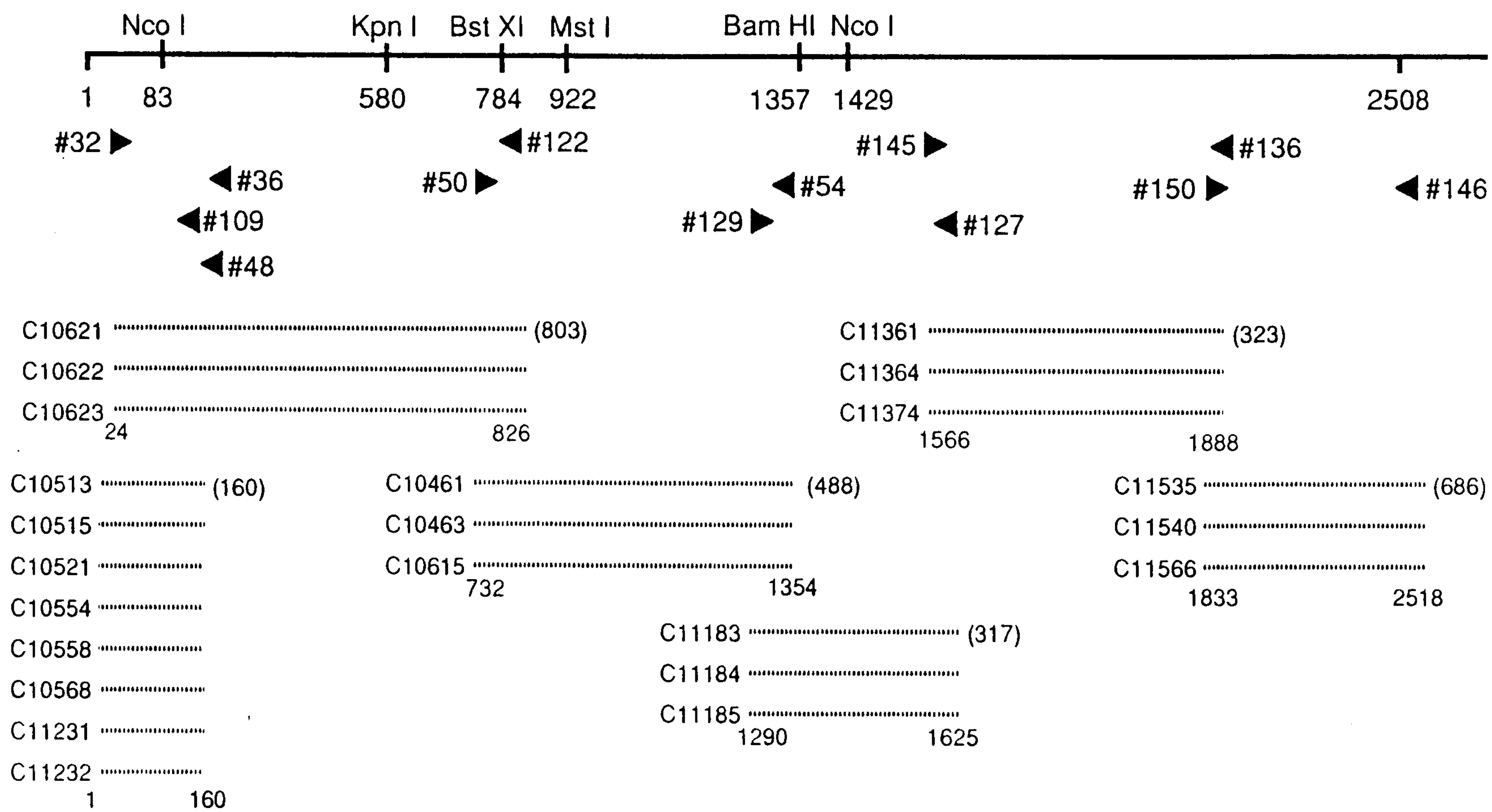

Nco I
Kpn I
Bst XI
Mst I
Bam HI
Nco I
1
83
580
784
922
1357
1429
2508
#32
#36
#109
#48
#50
#122
#129
#54
#145
#127
#136
#150
#146
C10621 (803)
C10622
C10623
24
826
C11361 (323)
C11364
C11374
1566
1888
C10513 (160)
C10515
C10521
C10554
C10558
C10568
C11231
C11232
1
160
C10461 (488)
C10463
C10615
732
1354
C11535 (686)
C11540
C11566
1833
2518
C11183 (317)
C11184
C11185
1290
1625

Fig. 6

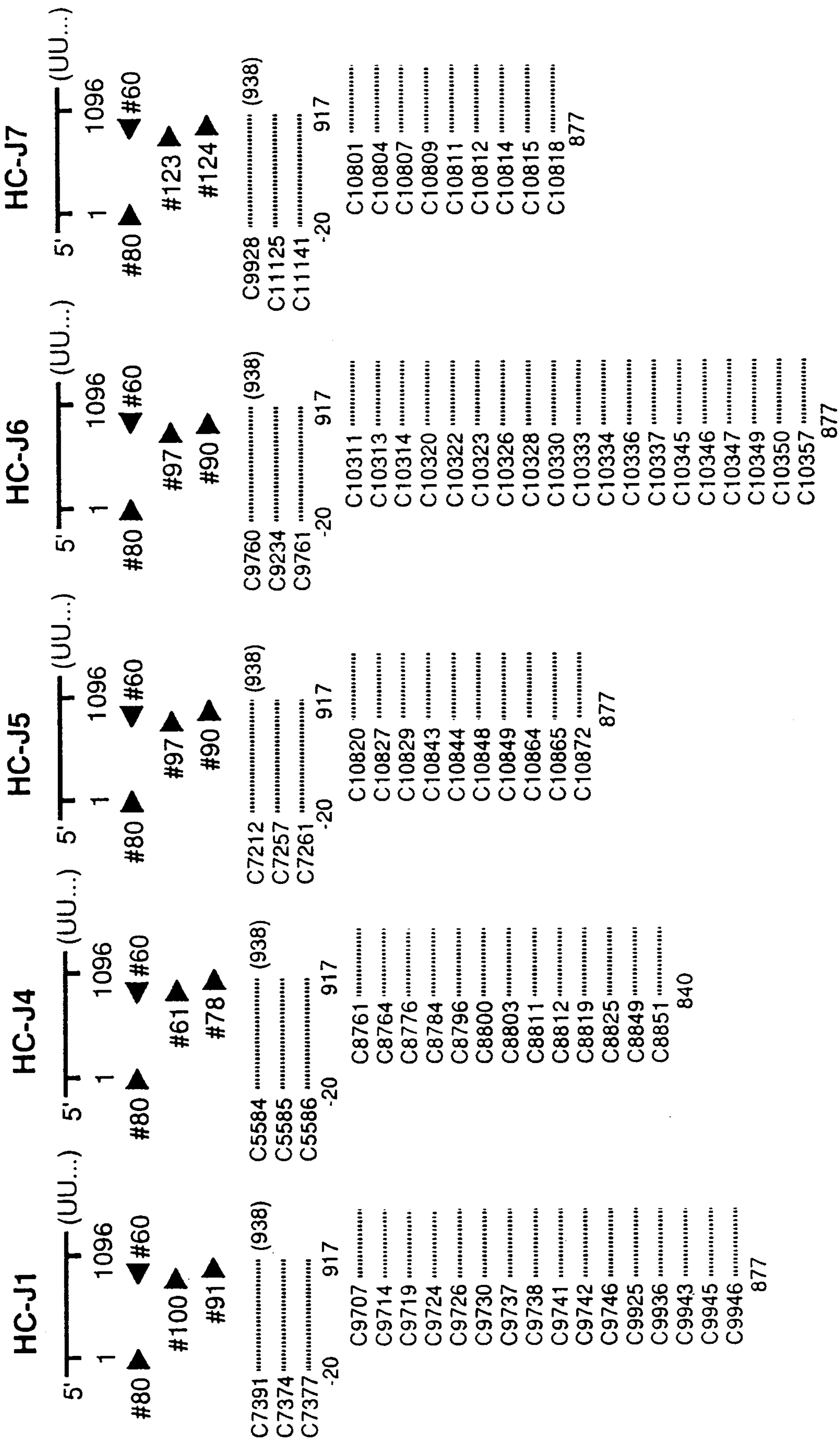
HC-J1
5'
1
1096
(UU...)
#80
#60
#100
#91
C7391 (938)
C7374
C7377
-20
917
C9707
C9714
C9719
C9724
C9726
C9730
C9737
C9738
C9741
C9742
C9746
C9925
C9936
C9943
C9945
C9946
877
HC-J4
5'
1
1096
(UU...)
#80
#60
#61
#78
C5584 (938)
C5585
C5586
-20
917
C8761
C8764
C8776
C8784
C8796
C8800
C8803
C8811
C8812
C8819
C8825
C8849
C8851
840
HC-J5
5'
1
1096
(UU...)
#80
#60
#97
#90
C7212 (938)
C7257
C7261
-20
917
C10820
C10827
C10829
C10843
C10844
C10848
C10849
C10864
C10865
C10872
877
HC-J6
5'
1
1096
(UU...)
#80
#60
#97
#90
C9760 (938)
C9234
C9761
-20
917
C10311
C10313
C10314
C10320
C10322
C10323
C10326
C10328
C10330
C10333
C10334
C10336
C10337
C10345
C10346
C10347
C10349
C10350
C10357
877
HC-J7
5'
1
1096
(UU...)
#80
#60
#123
#124
C9928 (938)
C11125
C11141
-20
917
C10801
C10804
C10807
C10809
C10811
C10812
C10814
C10815
C10818
877

European Patent Office

# EUROPEAN SEARCH REPORT

Application Number EP 92303625.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,A | EP - A - 0 461 863 (IMMUNO JAPAN INC.) * Totality * -- | 1,2, 10,12, 13 | C 07 H 21/04<br>C 12 Q 1/68<br>C 12 Q 1/70<br>C 12 N 15/51<br>C 12 P 19/34 |
| P,A | EP - A - 0 468 657 (TONEN CORPORATION) * Abstract; claims 1-18, 27-32 * -- | 2,10 | |
| P,A | PATENT ABSTRACTS OF JAPAN, unexamined applications, C section, vol. 16, no. 114, March 23, 1992 THE PATENT OFFICE JAPANESE GOVERNMENT page 13 C 921 * Kokai-no. 3-285 698 (KURARAY CO LTD.) * -- | 1,2,10 | |
| P,A | PATENT ABSTRACTS OF JAPAN, unexamined applications, C section, vol. 16, no. 114, March 23, 1992 THE PATENT OFFICE JAPANESE GOVERNMENT page 13 C 921 * Kokai-no. 3-285 699 (KURARAY CO LTD.) * -- | 1,2,10 | TECHNICAL FIELDS SEARCHED (Int. Cl.5)<br>C 07 H<br>C 12 Q<br>C 12 N<br>C 12 P |
| P,A | WO - A - 91/15 603 (GENELABS INCORPORATED) * Claims 13,16-18 * -- | 1,2,10 | |
| P,A | EP - A - 0 441 644 (TETSUO NAKAMURA) * Claims 1-4,9,20 * -- | 1,2,10 | |
| P,A | EP - A - 0 435 229 (SANWA KAGAKU KENKYUSHO CO.) * Abstract; claims * | 1,2,10 | |

The present search report has been drawn up for all claims

| Place of search | Date of completion of the search | Examiner |
|---|---|---|
| VIENNA | 09-07-1992 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

-2-
EP 92303625.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A - 0 398 748 (CHIRON CORPORATION) * Claims 1-18; fig. 18.1-18.11 * | 1,2,10 | |
| A | EP - A - 0 388 232 (CHIRON CORPORATION) * Claims 1,2,15,16,20 * | 1,2, 10,12, 13 | |
| A | EP - A - 0 318 216 (CHIRON CORPORATION) * Claims 1-7,21-23,29 * | 1,2, 10,12, 13 | |

TECHNICAL FIELDS SEARCHED (Int. Cl.5)

The present search report has been drawn up for all claims

| Place of search | Date of completion of the search | Examiner |
|---|---|---|
| VIENNA | 09-07-1992 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)